(19) Europäisches Patentamt
European Patent Office
Office européen des brevets

(11) Veröffentlichungsnummer: **0 102 925**
**B1**

(12) **EUROPÄISCHE PATENTSCHRIFT**

(45) Veröffentlichungstag der Patentschrift: **12.10.88**

(21) Anmeldenummer: **83810386.9**

(22) Anmeldetag: **26.08.83**

(51) Int. Cl.⁴: **C 07 D 251/16**, C 07 D 251/46, C 07 D 251/52, C 07 D 239/46, C 07 D 239/42, C 07 D 239/52, A 01 N 47/36 // C07C143/78, C07C143/828, C07C143/83, C07C143/833, C07C143/70

(54) **N-Phenylsulfonyl-N'-pyrimidinyl- und -triazinylharnstoffe.**

(30) Priorität: **01.09.82 CH 5201/82**

(43) Veröffentlichungstag der Anmeldung: **14.03.84 Patentblatt 84/11**

(45) Bekanntmachung des Hinweises auf die Patenterteilung: **12.10.88 Patentblatt 88/41**

(84) Benannte Vertragsstaaten: **AT BE CH DE FR GB IT LI NL SE**

(56) Entgegenhaltungen:
**EP-A-0 044 210**
**EP-A-0 044 807**
**EP-A-0 106 512**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Meyer, Willy, Talweg 49, CH- 4125 Riehen (CH)**
Erfinder: **Reinehr, Dieter, Dr., Wolfsheule 10, D-7842 Kandern (DE)**
Erfinder: **Oertle, Konrad, Dr., Vogesenstrasse 10, CH- 4106 Therwil (CH)**
Erfinder: **Schurter, Rolf, Dr., Holzmattstrasse 45, CH- 4102 Binningen (CH)**

**EP 0 102 925 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft neue, herbizid wirksame und pflanzenwuchsregulierende N-Phenylsulfonyl-N'-pyrimidinyl- und -triazinyl-harnstoffe, Verfahren zu ihrer Herstellung, sie als Wirkstoffe enthaltende Mittel, sowie deren Verwendung zum Bekämpfen von Unkräutern, vor allem selektiv in Nutzpflanzenkulturen oder zum Regulieren und Hemmen des Pflanzenwachstums.

Die erfindungsgemässen N-Phenylsulfonyl-N'-pyrimidinyl- und triazinyl-harnstoffe entsprechen der allgemeinen Formel I

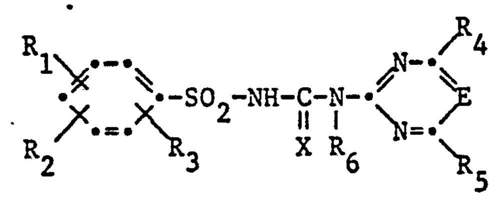 (I),

worin

$R_1$ Wasserstoff, Halogen, Nitro, Amino, $C_1$-$C_5$-Alkyl, $C_1$-$C_4$-Halogenalkyl oder einen Rest $-Q-R_7$, $-CO-OR_8$ oder $-(CO)_n-NR_9R_{10}$,

$R_2$ Wasserstoff, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Halogen oder Alkoxyalkyl mit höchstens 4 Kohlenstoffatomen,

$R_3$ einfach oder mehrfach durch Fluor substituiertes $C_2$-$C_{10}$-Alkenyl, dessen Doppelbindung direkt an den Phenylkern gebunden ist,

$R_4$ $C_1$-$C_3$-Alkyl, $C_1$-$C_3$-Halogenalkyl, $C_1$-$C_3$-Alkoxy oder $C_1$-$C_3$-Halogenalkoxy,

$R_5$ Wasserstoff Halogen $-NR_{13}R_{14}$, $C_1$-$C_3$-Alkyl, $C_1$-$C_3$-Halogenalkyl, $C_1$-$C_3$-Alkoxy oder $C_1$-$C_2$-Halogenalkoxy,

$R_6$ Wasserstoff, $C_1$-$C_3$-Alkyl oder $C_1$-$C_3$-Alkoxy,

X Sauerstoff oder Schwefel und

E Stickstoff oder die Methingruppe bedeuten, wobei

$R_7$ für gegebenenfalls durch Halogen oder $C_1$-$C_3$-Alkoxy substituiertes $C_1$-$C_4$-Alkyl oder $C_3$-$C_5$-Alkenyl,

$R_8$ für $C_1$-$C_4$-Alkyl, $C_1$-$C_4$-Halogenalkyl oder $C_2$-$C_6$-Alkoxyalkyl,

$R_9$, $R_{10}$, $R_{13}$ und $R_{14}$ unabhängig voneinander für Wasserstoff oder $C_1$-$C_3$-Alkyl,

Q für Sauerstoff, Schwefel, die Sulfinyl- oder Sulfonylbrücke, und

n für Null oder eins stehen,

sowie den Salzen dieser Verbindungen.

Harnstoffverbindungen, Triazinverbindungen und Pyrimidinverbindungen mit herbizider Wirkung sind allgemein bekannt. Kürzlich wurden Aryl-sulfamoyl-heterocyclyl-aminocarbamoylverbindungen mit herbizider und pflanzenwuchsregulierender Wirkung, beispielsweise in der EP-Patentanmeldung 44 210, in der DE-OS-2 715 786 oder im niederländischen Patent 121 788 beschrieben.

In den Definitionen ist unter Alkyl geradkettiges oder verzweigtes Alkyl zu verstehen; z. B.: Methyl, Äthyl, n-Propyl, i-Propyl, die vier isomeren Butyl, n-Amyl, i-Amyl, 2-Amyl, 3-Amyl, n-Hexyl oder i-Hexyl.

Unter Alkoxy ist zu verstehen: Methoxy, Äthoxy, n-Propoxy, i-Propoxy und die vier isomeren Butoxyreste, insbesondere aber Methoxy, Äthoxy oder i-Propoxy.

Beispiele für Alkylthio sind Methylthio, Acthylthio, n-Propylthio, i-Propylthio und n-Butylthio, insbesondere aber Methylthio und Äthylthio.

Beispiele für Alkenylreste sind Vinyl, Allyl, Isopropenyl, 1-Propenyl, 1-Butenyl, 2-Butenyl, 3-Butenyl, 1-Isobutenyl, 2-Isobutenyl, 1-Pentenyl, 2-Pentenyl, 3-Pentenyl und 4-Pentenyl, insbesondere aber Vinyl, Allyl und 4-Pentenyl.

Beispiele für Alkylsulfinyl sind Methylsulfinyl, Äthylsulfinyl, n-Propylsulfinyl und n-Butylsulfinyl, insbesondere aber Methylsulfinyl und Äthylsulfinyl.

Beispiel für Alkylsulfonyl sind Methylsulfonyl, Äthylsulfonyl, n-Propylsulfonyl und n-Butylsulfonyl, insbesondere aber Methylsulfonyl und Äthylsulfonyl.

Unter Halogen in den Definitionen sowie in Halogenalkyl, -alkoxy, -alkylsulfinyl, -alkylsulfonyl und -alkylthio sind Fluor, Chlor und Brom, vorzugsweise jedoch Fluor und Chlor zu verstehen.

Alkinylresten in den Definitionen der obigen Symbole entsprechen in der Regel Propargyl, 2-Butinyl, 3-Butinyl, sowie isomere Pentinyl- oder Hexinylreste, vorzugsweise ist der Alkinylrest jedoch durch Propargyl oder 2- oder 3-Butinyl verkörpert.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Äthylamin, Propylamin, i-Propylamin, die vier isomere Butylamine, Dimethylamin, Diäthylamin, Diäthanolamin, Dipropylamin, Diisopropylamin, Di-n-butylamin, Pyrrolidin,

Piperidin, Morpholin, Trimethylamin, Triäthylamin, Tripropylamin, Chinuclidin, Pyridin, Chinolin und i-Chinolin, insbesondere aber Äthyl-, Propyl-, Diäthyl- oder Triäthylamin, vor allem aber iso-Propylamin und Diäthanolamin.

Substituierte Alkenylreste, die der Definition des Substituenten $R_3$ der vorliegenden Erfindung entsprechen, sind beispielsweise: 3-Fluor-1-propen-1-yl, 1-Fluormethyl-vinyl, 1-Difluormethyl-vinyl, 1-Trifluormethyl-vinyl, 3,3-Difluor-1-propen-1-yl, 3,3,3-Trifluor-1-propen-1-yl, 2,2-Difluorvinyl, Perfluorvinyl oder 3-Fluor-1-buten-1-yl.

Bevorzugt sind Fluoralkenylreste mit höchstens 5 Kohlenstoffatomen. Solche bevorzugten Alkenylreste sind 3,3,3-Trifluor-1-propen-1-yl und 3,3-Difluor-1-propen-1-yl.

Beispiele für quaternäre Ammoniumbasen sind im allgemeinen die Kationen von Halogenammoniumsalzen, z. B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Von den erfindungsgemässen Verbindungen der Formel I sind die Verbindungen bevorzugt, in denen entweder

a) X Sauerstoff bedeutet oder
b) $R_4$ und $R_5$ zusammen nicht mehr als 4 Kohlenstoffatome enthalten oder
c) $R_6$ Wasserstoff bedeutet oder
d) $R_1$ und $R_2$ Wasserstoff bedeuten oder
e) $R_3$ 2 bis 4 Kohlenstoffatome enthält oder
f) $R_3$ die 2-Stellung zur Sulfonylgruppe besetzt.

Eine ganz besonders bevorzugte Untergruppe von Verbindungen der Formel I bilden diejenigen Verbindungen, in denen X für Sauerstoff und $R_1$, $R_2$ und $R_6$ für Wasserstoff stehen, $R_4$ und $R_5$ zusammen nicht mehr als 4 Kohlenstoffatome enthalten und $R_3$ die 2-Stellung zur Sulfonylgruppe besetzt und 2 bis 4 Kohlenstoffatome enthält.

Als bevorzugte Einzelverbindungen sind zu nennen:

N-[2-(3,3,3-Trifluor-1-propen-1-yl)-phenyl-sulfonyl]-N'-(4-methoxy-6-methyl-1,3,5-triazin-2-yl)-harnstoff und

N-[2-(3,3,3-Trifluor-1-propen-1-yl)-phenyl-sulfonyl]-N'-(4,6-dimethoxy-1,3,5-triazin-2-yl)-harnstoff.

Die Herstellung der Verbindungen der Formel I erfolgt in einem inerten, organischen Lösungsmittel.

Nach einem ersten Verfahren werden die Verbindungen der Formel I erhalten, indem man ein Phenylsulfonamid der Formel II

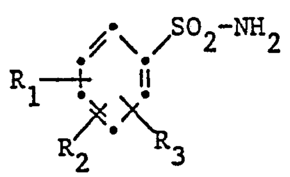
(II),

worin $R_1$, $R_2$ und $R_3$ die unter Formel I gegebene Bedeutung haben, in Gegenwart einer Base mit einem N-Pyrimidinyl- oder -Triazinylcarbamat der Formel III

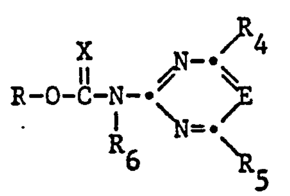
(III),

worin E, $R_4$, $R_5$, $R_6$ und X die unter Formel I gegebene Bedeutung haben und R für Phenyl, Alkyl oder substituiertes Phenyl steht, umsetzt.

Nach einem weiteren Verfahren gelangt man zu Verbindungen der Formel I, indem man ein Phenylsulfonylisocyanat oder -isothiocyanat der Formel IV

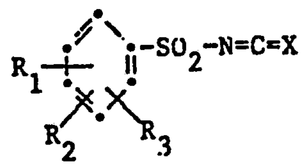

(IV),

worin $R_1$, $R_2$, $R_3$ und X die unter Formel I gegebene Bedeutung haben, gegebenenfalls in Gegenwart einer Base, mit einem Amin der Formel V

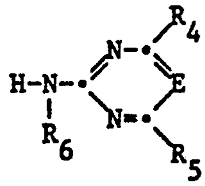

(V),

worin E, $R_4$, $R_5$ und $R_6$ die unter Formel I gegebene Bedeutung hagen, umsetzt.

Nach einem weiteren Verfahren werden die Verbindungen der Formel I hergestellt, indem man ein Sulfonamid der oben angegebenen Formel II gegebenenfalls in Gegenwart einer Base mit einem Isocyanat oder Isothiocyanat der Formel VI

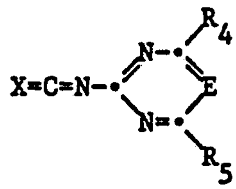

(VI),

worin E, $R_4$, $R_5$ und X unter Formel I gegebene Bedeutung haben, umsetzt.

Schließlich kann man die Verbindungen der Formel I auch erhalten, indem man ein N-Phenylsulfonylcarbamat der Formel VII

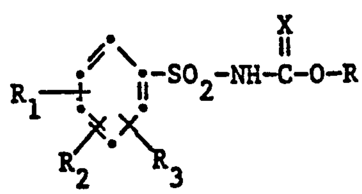

(VII),

worin $R_1$, $R_2$, $R_3$ und X die unter Formel I angegebene Bedeutung haben und R für Phenyl, Alkyl oder substituiertes Phenyl steht, mit einem Amin der oben angegebenen Formel V umsetzt.

Die erhaltenen Harnstoffe der Formel I können gewünschtenfalls mittels Aminen, Alkalimetall- oder Erdalkalimetallhydroxiden oder quaternären Ammoniumbasen in Additionssalze übergeführt werden. Dieses geschieht beispielsweise durch Umsetzen mit der äquimolaren Menge Base und Verdampfen des Lösungsmittels.

Die Umsetzungen zu Verbindungen der Formel I werden vorteilhafterweise in aprotischen, inerten, organischen Lösungsmitteln vorgenommen wie Methylenchlorid, Tetrahydrofuran, Acetonitril, Dioxan, Toluol.

Die Reaktionstemperaturen liegen vorzugsweise zwischen -20° und +120°C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base oder Isocyanat als Reaktionskatalysator verkürzt werden.

Die Endprodukte können durch Einengen und/oder Verdampfen des Lösungsmittels isoliert und durch Umkristallisieren oder Zerreiben des festen Rückstandes in Lösungsmitteln, in denen sie sich nicht gut lösen, wie Äther, aromatischen Kohlenwasserstoffen oder chlorierten Kohlenwasserstoffen, gereinigt werden.

Die Wirkstoffe der Formel I sind stabile Verbindungen. Ihre Handhabung bedarf keiner vorsorglichen Massnahmen.

Die Ausgangsstoffe der Formeln II, IV und VII sind zum Teil neu. Sie können nach der folgenden Methode hergestellt werden.

So können beispielsweise die neuen Zwischenprodukte (vgl. Teilanmeldung Nr. EP-A-248 245) der engeren Formel IIa

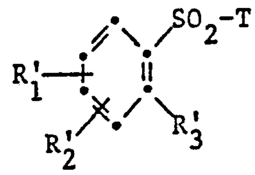

(IIa),

worin

T Hydroxyl, -OM, $-O\frac{M_1}{2}$, Cl, $-N=C=O$ oder $-NHT_4$, M ein Alkalimetallatom, $M_1$ ein Erdalkalimetallatom,

$R'_1$ Wasserstoff, Halogen, $C_1$-$C_5$-Alkyl, $C_1$-$C_4$-Halogenalkyl, $C_1$-$C_4$-Alkoxy, $-COOR_8$, $-CONR_9R_{19}$ oder $-NO_2$,

$R'_2$ Wasserstoff, Halogen $C_1$-$C_4$-Alkyl, $C_1$-$C_4$-Halogenalkyl, $C_1$-$C_4$-Alkoxy, $C_1$-$C_4$-Halogenalkoxy oder Alkoxyalkyl mit höchstens 4 Kohlenstoffatomen und

$R'_3$ eine Gruppe $-C(T_1)=C(T_2)T_3)$ bedeuten,

$T_1$ Wasserstoff oder $C_1$-$C_4$-Alkyl,

$T_2$ ein- oder mehrfach durch Fluor substituiertes $C_1$-$C_8$-Alkyl,

$T_3$ Wasserstoff oder gegebenenfalls durch Fluor substituiertes $C_1$-$C_5$-Alkyl, und

$T_4$ Wasserstoff, -CO-NH-$C_1$-$C_4$-Alkyl, -COO-$C_1$-$C_4$-Alkyl oder -COO-Phenyl bedeuten, wobei durch $T_1$, $T_2$ und $T_3$ dargestellte Alkylgruppen zusammen höchstens 8 Kohlenstoffatome aufweisen,

dadurch hergestellt werden, daß man ein Amin der Formel VIII

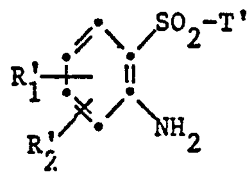

(VIII)

zu einem Diazoniumsalz der Formel IX

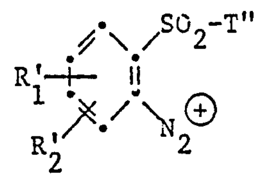

(IX)

diazotiert, dieses in Gegenwart von Palladium-Katalysatoren, die unter den Reaktionsbedingungen Pd(O)-Verbindungen bilden, und gegebenenfalls in Gegenwart einer Base mit einer Verbindung der Formel X

$$H - R'_3 \qquad (X)$$

zu einer Verbindung der Formel XI

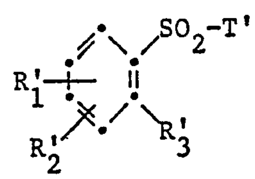

(XI)

umsetzt, die Verbindung der Formel XI in an sich bekannter Weise durch Behandlung mit einem Chlorierungsmittel, wie Thionylchlorid oder $PCl_5$ in das entsprechende Sulfonylchlorid überführt, dieses mit Ammoniak behandelt und so erhaltene Verbindungen der Formel IIa, worin T für $-NH_2$ steht, gegebenenfalls in Verbindungen der Formel IIa, worin $T_4$ nicht Wasserstoff bedeutet, überführt, z. B. durch Umsetzung mit den Acylierungsmitteln $O=C=N$-$C_1$-$C_4$-Alkyl, ClCONH-$C_1$-$C_4$-Alkyl, ClCOO-$C_1$-$C_4$-Alkyl, ClCOO-Phenyl oder in Gegenwart von $C_1$-$C_4$-Alkylisocyanaten, wobei dann T' OH, OM oder $O\frac{M_1}{2}$ und T'' OM oder $O\frac{M_1}{2}$ bedeutet und M, $M_1$, $R'_1$, $R'_2$, $R'_3$ die unter Formel IIa angegebene Bedeutung haben.

Die Diazotierung kann nach an sich bekannten Methoden in saurem wässrigem Medium, wie wäßriger HCl, $H_2SO_4$, $H_2O$/Essigsäure, durchgeführt werden. Die Umsetzung der Diazoniumsalze mit den Olefinen wird zweckmäßig in Gegenwart eines inerten organischen Lösungsmittels vorgenommen. Dazu eignen sich z. B. gegebenenfalls chlorierte aliphatische Monocarbonsäuren, besonders Essigsäure, Chloressigsäure, Dichloressigsäure oder Trifluoressigsäure, Aceton, Dichlormethan und Acetonitril oder Gemische der genannten Lösungsmittel. Bevorzugt verwendet man Essigsäure.

Als Palladium-Katalysatoren und Basen können z. B. solche der in der EP-Patentanmeldung 40 177

beschriebenen Art verwendet werden. Bevorzugte Palladium-Katalysatoren sind $PdCl_2$, $[PdCl_4]Na_2$ oder $[PdCl_4]Li_2$, vor allem jedoch Bis-(dibenzylidenaceton)Palladium(O). Als Basen verwendet man vorzugsweise Alkalimetallcarboxylate, wie Natriumacetat. Eine Isolierung der Diazoniumsalze der Formel IX und der Sulfonylchloride ist im allgemeinen nicht erforderlich. Wird die Diazotierung in Gegenwart von Essigsäure und von nur einem Äquivalent einer starken Säure durchgeführt und werden die erhaltenen Diazoniumsalze ohne Isolierung weiterverwendet, so kann im allgemeinen bei der Umsetzung mit den Olefinen der Formel X auf den Zusatz von Base verzichtet werden.

Verbindungen der Formel IIa, worin $R'_1$ und $R'_2$ ungleich Brom bzw. Jod sind, können auch dadurch hergestellt werden, daß man eine Verbindung der Formel XII

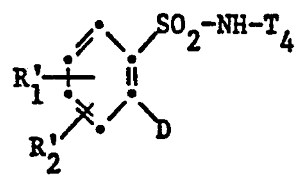

(XII)

in Gegenwart einer gegebenenfalls arsen- oder phosphorhaltigen Palladium-Verbindung als Katalysator und einer Base mit einem Olefin der Formel X umsetzt, worin $R'_1$, $R'_2$ und $T_4$ die unter Formel IIa angegebene Bedeutung haben, D Brom oder Jod bedeutet.

Als Palladium-Katalysatoren und Basen für diese Herstellungsvariante eignen sich beispielsweise Verbindungen der in der US-PS-3 922 299 beschriebenen Art. Als Katalysatoren verwendet man bevorzugt Gemische aus Palladiumacetat und Triphenylphosphin oder Tri-o-tolylphosphin. Stellt D in Formel XII Jod dar, so können auch arsen- bzw. phosphorfreie Palladium-Verbindungen, vor allem Palladiumacetat, eingesetzt werden. Als Basen eignen sich vor allem Trialkylamine, besonders Triäthylamin oder Tri-n-butylamin, und Alkalimetallcarboxylate, vor allem Natriumacetat.

Die Palladiumkatalysatoren werden bei beiden Verfahrensvarianten zweckmäßig in einer Menge von etwa 0,01 bis 5 Mol-%, bezogen auf das Diazoniumsalz der Formel IX bzw. das Halogenbenzol der Formel XII, verwendet.

Die Umsetzung der Halogenbenzole der Formel XII mit den Olefinen der Formel X wird ebenfalls zweckmäßig in Gegenwart eines inerten organischen Lösungsmittels vorgenommen, z. B. gegebenenfalls chlorierten aromatischen Kohlenwasserstoffen, wie Toluol, Xylolen oder Chlorbenzol, oder N,N-Dialkylamiden von aliphatischen Monocarbonsäuren der oben erwähnten Art, besonders N,N-Dimethylformamid.

Die übrigen neuen Zwischenprodukte der Formel II können prinzipiell nach zwei unterschiedlichen Syntheseprinzipien erhalten werden, die in Anlehnung an bekannte Verfahren durchgeführt werden.

So erhält man die Sulfonamide der Formel II aus entsprechenden Benzosulfonamiden durch Einführung der Alkenylseitenkette, bzw. Modifikation einer vorhandenen Seitenkette nach bekannten Verfahren. Analoge Verfahren sind in der EP-A-44 210 beschrieben.

Weiter erhält man die Sulfonamide der Formel II, indem man entsprechende substituierte Aniline diazotiert und mit Schwefeldioxid und Ammoniak in die Sulfonamide überführt. Analoge Verfahren sind in der EP-A-44 807 publiziert.

Andere Sulfonamide der Formel II erhält man, indem man entsprechend substituierte Thiobenzyläther chloriert und die entandenen Sulfochloride mit Ammoniak versetzt. Analoge Verfahren sind in der EP-A-41 404 publiziert.

Die Ausgangsprodukte der Formeln III, V, VI, VIII und XII sind bekannt oder können auf an sich bekannte Weise hergestellt werden.

Die Verbindungen der Formeln IV und VII können in an sich bekannter Weise aus den Verbindungen der Formel II bzw. IIa erhalten werden.

So können die Phenylsulfonylisocyanate der Formel IV durch Umsetzungen der Sulfonamide der Formel II mit Phosgen in Anwesenheit von Butylisocyanat in einem chlorierten Kohlenwasserstoff als Lösungsmittel, bei Rückflusstemperatur erhalten werden. Ähnliche Darstellungen sind in "Neuere Methoden der präparativen organischen Chemie", Band VI, 211 - 229, Verlag Chemie, Weinheim, 1970, beschrieben.

Die Isothiocyanate der Formel IV werden durch Behandlung der Sulfonamide der Formel II mit Schwefelkohlenstoff und Kaliumhydroxid und anschliessender Umsetzung des Dikaliumsalzes mit Phosgen erhalten. Solche Verfahren sind in Arch. Pharm. 299, 174 (1966) beschrieben. Die N-Phenylsulfonylcarbamate der Formel VII werden durch Umsetzung der Sulfonamide der Formel II mit Diphenylcarbonat in Gegenwart einer Base erhalten. Ähnliche Verfahren sind in der japanischen Patentschrift 61 169 erwähnt.

Neue Verbindungen der Formeln III und VI können nach bekannten Methoden aus entsprechenden Verbindungen der Formel V erhalten werden.

Neue Fluoralkoxy-aminopyrimidine und -triazine der Formel V und ihre Herstellung sowie die Herstellung entsprechender Verbindungen der Formel III und VI daraus werden in der EP-A-70 804 beschrieben.

Durch Umsetzung von Aminen der Formel V mit Oxalylchlorid in chlorierten Kohlenwasserstoffen als Lösungsmittel, lassen sich Isocyanate der Formel VI herstellen. Amine der Formel V sind bekannt und zum Teil im Handel erhältlich oder sie können nach bekannten Methoden hergestellt werden, siehe "The Chemistry of Heterocyclic Compounds" Band XIV, Interscience Publishers, New York, London.

Die Verbindungen der Formel IIa sind neu und speziell für die Synthese der Wirkstoffe der Formel I entwickelt worden.

In Formel IIa stellt bevorzugt mindestens eines von $T_1$ und $T_3$, insbesondere $T_1$, Wasserstoff dar. T ist vorzugsweise $-NH_2$. Im übrigen gelten für $R'_1$, $R'_2$ und $R'_3$ die unter Formel I für $R_1$, $R_2$ und $R_3$ angegebenen bevorzugten Bedeutungen.

Bei geringeren Aufwandmengen zeichnen sich die Verbindungen der Formel I durch gute selektivwuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Die bevorzugte Kultur ist Getreide wie Weizen, Gerste und Roggen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen Herbiziden und Wuchsregulatoren - sehr geringen Aufwandmengen.

Die Verbindungen der Formel I haben ausserdem starke pflanzenwuchsregulierende, insbesondere pflanzenwuchshemmende, Eigenschaften. Es werden sowohl Monokotyledonen als auch Dikotyledonen in ihrem Wachstum beeinträchtigt.

Eine Hemmung des vegetativen Wachstums erlaubt bei vielen Kulturpflanzen eine dichtere Anpflanzung der Kultur, so daß ein Mehrertrag, bezogen auf die Bodenfläche, erzielt werden kann.

Ein weiterer Mechanismus der Ertragsteigerung mit Wachstumshemmern beruht darauf, daß die Nährstoffe in stärkerem Masse der Blüten- und Fruchtbildung zugute kommen, während das vegetative Wachstum eingeschränkt wird.

So können z. B. die in der Landwirtschaft in tropischen Gegenden häufig als "cover crops" (Bodenbedecker) angepflanzten Leguminosen durch die Verbindungen der Formel I in ihrem Wachstum selektiv gehemmt werden, so daß zwar die Bodenerosion zwischen den Kulturpflanzen verhindert wird, die "cover crops" jedoch nicht zur Konkurrenz für die Kultur werden können.

Weiter eignen sich die Verbindungen der Formel I um das Keimen von eingelagerten Kartoffeln zu verhindern. Bei Kartoffeln entwickeln sich bei der Einlagerung über den Winter häufig Keime, die Schrumpfen, Gewichtsverlust und Faulen zur Folge haben.

Bei größeren Aufwandmengen werden alle getesteten Pflanzen in ihrer Entwicklung so geschädigt, daß sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Regulierung des Pflanzenwuchses von monokotylen und dikotylen Pflanzen, insbesondere Gräsern, Nutzpflanzen, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z. B. zu Emulsionskonzentraten, direkt versprühbaren oder verdünnbaren Lösungen, werdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z. B. für Stäubemittel und dispergierbare pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorganulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche

synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z. B. die Na- oder K-Salze der Öl- oder Stearinsäure, oder von natürlichen Fettsäuregemischen, die z. B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2-Sulfonsäuregruppen und einen Fettsäurerest mit 8 - 22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Äthylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl oder niedrige Hydroxyalkylreste aufweisen. Die Salze liegen vorzugsweise als Halogenide, Methylsulfate uder Äthylsulfate vor, z. B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Rigdewood, New Jersey, 1981;

H. Stache, "Tensid-Taschenbuch", 2. Aufl., C. Hanser Verlag, München, Wien, 1981;

M. and J. Ash. "Encyclopedia of Surfactants", Vol. I-III, Chemical Publishing Co., New York, 1980 - 1981.

Die pestiziden Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent)

**Emulgierbare Konzentrate**

| | |
|---|---|
| Aktiver Wirkstoff: | 1 bis 20 %, bevorzugt 5 bis 10 % |
| oberflächenaktives Mittel: | 5 bis 30 %, vorzugsweise 10 bis 20 % |
| flüssiges Trägermittel: | 50 bis 94 %, vorzugsweise 70 bis 85 %. |

**Stäube**

| | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 %. |

**Suspension-Konzentrate**

| | |
|---|---|
| Aktiver Wirkstoff: | 5 bis 75 %, vorzugsweise 10 bis 50 % |
| Wasser: | 94 bis 25 %, vorzugsweise 90 bis 30 % |
| oberflächenaktives Mittel: | 1 bis 40 %, vorzugsweise 2 bis 30 %. |

**Benetzbare Pulver**

| | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermittel: | 5 bis 95 %, vorzugsweise 15 bis 90 %. |

**Granulate**

| | |
|---|---|
| Aktiver Wirkstoff: | 0,5 bis 30 %, vorzugsweise 3 bis 15 % |
| festes Trägermittel: | 99,5 bis 70 % vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C, die Drücke in Millibar mb angegeben.

**Herstellungsbeispiele:**

**Beispiel 1:** N-[2-(3,3,3-Trifluor-1-propen-1-yl)-phenyl-sulfonyl]-N'-(4-methoxy-6-methyl-1,3,5-triazin-2-yl)-harnstoff.

Eine Lösung von 5,0 g 2-(3,3,3-Trifluor-1-propen-1-yl)-phenylsulfonamid und 3,3 g 1,5-Diazabicyclo[5,4,0]undec-5-en in 80 ml Dioxan wird mit 5,2 g N-(4-Methoxy-6-methyl-1,3,5-triazin-2-yl)-phenylcarbonat versetzt und für 3 Stunden bei 20° bis 25°C gerührt. Anschliessend wird die klare Reaktionslösung in 300 ml Wasser aufgenommen und mit 2 N Salzsäure bis auf pH 4 - 5 angesäuert. Der ausgefallene harzartige Niederschlag wird mit Äthylacetat aus der wäßrigen Phase extrahiert. Der organische Extrakt wird getrocknet und eingedampft. Der ölige Rückstand wird aus Aceton/Äther-Gemisch (1 : 10) kristallisiert. Man erhält so 7,2 g N-[2-(3,3,3-Trifluor-1-propen-1-yl)-phenyl-sulfonyl]-N'-(4-methoxy-6-methyl-1,3,5-triazin-2-yl)-harnstoff, Smp. 159-160°C.

**Beispiel 2:** 2-(3,3,3-Trifluor-1-propen-1-yl)-phenylsulfonamid.

**a)** Orthanilsäure-Diazoniumsalz.

34,64 g (0,2 Mol) Orthanilsaure werden in 30 ml Wasser aufgeschlämmt, mit 62,3 ml Borfluorwasserstoffsäure (50 %-ig; 0,5 Mol) versetzt und auf 0 - 5°C gekühlt. Innerhalb einer Stunde werden bei dieser Temperatur tropfenweise 13,8 g (0,2 Mol) Natriumnitrit, gelöst in 20 ml Wasser, unter Rühren zugetropft. Nach zusätzlichem Rühren während 30 Minuten werden 100 ml Diäthyläther zugegeben und die abgekühlte Suspension wird filtriert. Das isolierte feste Diazoniumsalz wird zusätzlich mit 100 ml eines 1 : 1 Gemisches aus Essigsäure und Diäthyläther und anschließend mit nochmals 100 ml Diäthyläther gewaschen. Nach kurzem Trocknen an der Luft erhält man 34 g Diazoniumsalz; Ausbeute 92 % d.Th. Ohne Ausbeuteeinbusse kann anstelle von Borfluorwasserstoffsäure konz. Salzsäure oder konz. Schwefelsäure verwendet werden.

**b)** 8,06 g (0,0438 Mol) des Diazoniumsalzes der Orthanilsäure werden in 150 ml Essigsäure aufgeschlämmt und mit 3,59 g (0,0438 Mol) Natriumacetat versetzt. Nach Zugabe von 0,256 g (4,38 x $10^{-4}$ Mol) Bis-(benzylidenaceton)Palladium(O) wird das Reaktionsgefäß (250 ml-Fischer-Porter-Flasche/Druckapparatur) einmal evakuiert. Dann werden 6 g (0,0625 Mol) 3,3,3-Trifluorpropen aufgepreßt. Unter starkem Rühren setzt

die Reaktion sofort unter Stickstoffentwicklung (Druckanstieg bis ca. 8 bar) und Temperaturanstieg (max. ca. 45° C) ein und ist nach ca. 90 Minuten beendet. Das Lösungsmittel wird im Rotationsverdampfer abgezogen.

Der erhaltene Rückstand (9,96 g) wird in 50 ml N,N-Dimethylformamid gelöst und tropfenweise mit 6,61 ml (0,0909 Mol) Thionylchlorid versetzt. Nach 2 Stunden Rühren bei Raumtemperatur wird das Reaktionsgemisch auf Eis gegossen und das entstandene Sulfonylchlorid wird abfiltriert. Man erhält 6,37 g Sulfonylchlorid, die direkt in 20 ml Essigsäureäthylester gelöst und bei 0 - 5° C in 25 ml konz. Ammoniak eingetropft werden. Nach vollständiger Umsetzung des Sulfonylchlorids wird mit Wasser verdünnt und mehrmals mit Essigsäureäthylester extrahiert. Aus den getrockneten Essigsäureäthylester-Phasen werden 5,35 g Produkt isoliert, das durch Chromatographie an Kieselgel oder durch Umkristallisieren aus Essigsäureäthylester/n-Hexan gereinigt wird. Man erhält 5,08 g (0 0239 Mol) 2-(3,3,3-Trifluor-1-propen-1-yl)-phenylsulfonamid; Ausbeute 54 % d.Th., bezogen auf das Diazoniumsalz, Smp. 153 - 154° C.

| | | | | | |
|---|---|---|---|---|---|
| Analyse: | $C_9H_8F_3NO_2S$ (251,22) | | | | |
| Ber. | C 43,03 | H 3,21 | F 22,69 | N 5,58 | S 12,76 |
| Gef. | C 42,99 | H 3,29 | F 22,73 | N 5,53 | S 12,97. |

**Beispiel 3:** 2-(2-Perfluorhexyl-vinyl)-phenylsulfonamid.

16,4 g (0,089 Mol) des Diazoniumsalzes der Orthanilsäure werden in 100 ml Essigsäure aufgeschlämmt. Dazu gibt man 7,31 g (0,089 Mol) Natriumacetat, gefolgt von 0,5116 g (8,9 x $10^{-4}$ Mol) Bis-(benzylidenaceton)Palladium(O). Bei Raumtemperatur läßt man 34,61 g (0,098 Mol)Perfluorhexyläthylen (85 %-ig) zutropfen. Unter $N_2$-Entwicklung setzt sofort die exotherme Reaktion ein. Die Reaktionstemperatur wird durch externe Kühlung und Änderung der Tropfgeschwindigkeit auf 30 - 40° C gehalten. Nach Beendigung der Reaktion wird die Essigsäure anschliessend am Rotationsverdampfer unter Zusatz von Toluol möglichst vollständig entfernt. Der erhaltene Rückstand wird wie in Beispiel 4 beschrieben ohne Reinigung über das Sulfonylchlorid in das entsprechende Sulfonamid übergeführt. Nach Chromatographie an Kieselgel erhält man 30,28 g (0,059 Mol) 2-(2-Perfluorhexylvinyl)-phenylsulfonamid in einer Ausbeute von 67 % d.Th. (bezogen auf das Diazoniumsalz); Smp. 60 - 61° C.

**Beispiel 4:** N-[2-(3,3,3-Trifluor-1-propen-1-yl)-phenyl-sulfonyl]-N'-(4-methoxy-6-methyl-1,3,5-triazin-2-yl)-harnstoff.

**a)** 5,0 g 2-(3,3,3,-Trifluor-1-propen-1-yl)-phenyl-sulfonamid und 1,7 g Methylisocyanat werden in 25 ml Methylenchlorid suspendiert und tropfenweise innerhalb von 10 Minuten mit 3,0 g Triäthylamin versetzt. Es entsteht eine klare Lösung. Nach dem Eindampfen der Lösung wird der Rückstand in 5 %-iger Natriumcarbonatlösung gelöst und von den unlöslichen Bestandteilen abfiltriert. Durch Ansäuern der klaren Lösung mit 10 %-iger Salzsäure erhält man als farblosen Niederschlag 5,9 g N-[2-(3,3,3-Trifluor-1-propen-1-yl)-phenyl-sulfonyl]-N'-methyl-harnstoff, Smp. 190 - 192° C.

**b)** 5,9 g N-[2-(3,3,3-Trifluor-1-propen-1-yl)-phenyl-sulfonyl]-N'-methyl-harnstoff werden in 100 ml Chlorbenzol suspendiert. Durch Rückflußkochen am Wasserabscheider wird die Lösung getrocknet. Anschliessend leitet man bei einer Temperatur von 120 - 130° C innerhalb von 20 Minuten 6,0 g Phosgen in das Reaktionsgemisch ein. Durch Abdampfen des Lösungsmittels erhält man als gelbliches Öl 5,6 g 2-(3,3,3-Trifluor-1-propen-1-yl)-phenyl-sulfonylisocyanat.

**c)** 5,6 g 2-(3,3,3-Trifluor-1-propen-1-yl)-phenyl-sulfonylisocyanat und 2,5 g 2-Amino-4-methoxy-6-methyl-1,3,5-triazin werden in 60 ml absolutem Dioxan für 3 Stunden bei 70 - 80° C gerührt. Nach dem Abkühlen auf 20° C wird das Reaktionsgemisch filtriert und die erhaltene klare Lösung auf ein Viertel des Volumens eingedampft. Nach der Zugabe von 50 ml Äther kristallisieren aus der Lösung 5,0 g N-[2-(3,3,3-Trifluor-1-propen-1-yl)-phenyl-sulfonyl]-N'-(4-methoxy-6-methyl-1,3,5-triazin-2-yl)-harnstoff aus, Smp. 154 - 155° C.

In analoger Weise erhält man die in der anschliessenden Tabelle aufgelisteten Zwischen- und Endprodukte.

**Tabelle 1:**

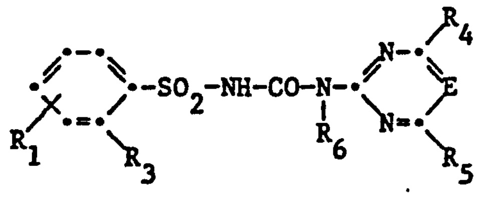

| Nr. | $R_1$ | $R_3$ | $R_4$ | $R_5$ | $R_6$ | E | phys.Daten |
|---|---|---|---|---|---|---|---|
| 1.1 | H | $-CH=CH-CF_3$ | $CH_3$ | $OCH_3$ | H | N | Smp. 159-160°C |
| 1.2 | H | $-CH=CH-CF_3$ | $OCH_3$ | $OCH_3$ | H | N | Smp. 185-186°C |
| 1.3 | H | $-CH=CH-CF_3$ | $CH_3$ | $OCH_3$ | H | CH | Smp. 166-169°C |
| 1.4 | H | $-CH=CH-CF_3$ | $CH_3$ | $CH_3$ | H | CH | Smp. 186-189°C |
| 1.5 | H | $-CH=CH-CF_3$ | $CH_3$ | $OCHF_2$ | H | CH | Smp. 178-179°C |
| 1.6 | H | $-CH=CH-CF_3$ | $OCH_3$ | $OCH_3$ | H | CH | Smp. 185-186°C |
| 1.7 | H | $-CH=CH-CF_3$ | $OCH_3$ | $-OCH_2-CF_3$ | H | N | Smp. 151-152°C |
| 1.8 | H | $-CH=CH-CF_3$ | $OCH_3$ | $-N(CH_3)_2$ | H | N | Smp. 174-175°C |
| 1.9 | H | $-CH=CH-CF_3$ | $OCH_3$ | $-N(CH_3)_2$ | H | CH | |
| 1.10 | H | $-CH=CH-CF_3$ | Cl | $OCHF_2$ | H | CH | |
| 1.11 | H | $-CH=CH-CF_3$ | $OCHF_2$ | $-N(CH_3)_2$ | H | CH | |
| 1.12 | H | $-CH=CH-CF_3$ | Cl | $OCH_3$ | H | CH | Smp. 174-175°C |
| 1.13 | H | $-CH=CH-CF_3$ | $OCH_3$ | $OCH_3$ | $CH_3$ | CH | |
| 1.14 | H | $-CH=CH-CH_2F$ | $CH_3$ | $CH_3$ | H | CH | |
| 1.15 | H | $-CH=CH-CH_2F$ | $CH_3$ | $OCH_3$ | H | CH | |
| 1.16 | H | $-CH=CH-CH_2F$ | $OCH_3$ | $OCH_3$ | H | CH | |
| 1.17 | H | $-CH=CH-CH_2F$ | $CH_3$ | $OCH_3$ | H | N | |
| 1.18 | H | $-CH=CH-CH_2F$ | $OCH_3$ | $OCH_3$ | H | N | |
| 1.19 | H | $-CH=CH-CHF_2$ | $CH_3$ | $CH_3$ | H | CH | |
| 1.20 | H | $-CH=CH-CHF_2$ | $CH_3$ | $OCH_3$ | H | CH | |
| 1.21 | H | $-CH=CH-CHF_2$ | $OCH_3$ | $OCH_3$ | H | CH | |
| 1.22 | H | $-CH=CH-CHF_2$ | $CH_3$ | $OCH_3$ | H | N | |
| 1.23 | H | $-CH=CH-CHF_2$ | $OCH_3$ | $OCH_3$ | H | N | |
| 1.24 | H | $-CH=CF_2$ | $CH_3$ | $CH_3$ | H | CH | |
| 1.25 | H | $-CH=CF_2$ | $CH_3$ | $OCH_3$ | H | CH | |
| 1.26 | H | $-CH=CF_2$ | $OCH_3$ | $OCH_3$ | H | CH | |
| 1.27 | H | $-CH=CF_2$ | $CH_3$ | $OCH_3$ | H | N | |
| 1.28 | H | $-CH=CF_2$ | $OCH_3$ | $OCH_3$ | H | N | |
| 1.29 | 6-F | $-CH=CH-CF_3$ | $CH_3$ | $CH_3$ | H | CH | |
| 1.30 | 6-F | $-CH=CH-CF_3$ | $CH_3$ | $OCH_3$ | H | CH | |
| 1.31 | 6-F | $-CH=CH-CF_3$ | $OCH_3$ | $OCH_3$ | H | CH | |
| 1.32 | 6-F | $-CH=CH-CF_3$ | $CH_3$ | $OCH_3$ | H | N | |
| 1.33 | 6-F | $-CH=CH-CF_3$ | $OCH_3$ | $OCH_3$ | H | N | |
| 1.34 | 5-F | $-CH=CH-CF_3$ | $CH_3$ | $CH_3$ | H | CH | |
| 1.35 | 5-F | $-CH=CH-CF_3$ | $OCH_3$ | $OCH_3$ | H | CH | |
| 1.36 | 5-F | $-CH=CH-CF_3$ | $CH_3$ | $OCH_3$ | H | N | |
| 1.37 | 5-F | $-CH=CH-CF_3$ | $OCH_3$ | $OCH_3$ | H | N | |
| 1.38 | 3-F | $-CH=CH-CF_3$ | $CH_3$ | $CH_3$ | H | CH | |
| 1.39 | 3-F | $-CH=CH-CF_3$ | $CH_3$ | $OCH_3$ | H | CH | |
| 1.40 | 3-F | $-CH=CH-CF_3$ | $OCH_3$ | $OCH_3$ | H | CH | |

| | | | | | | | |
|---|---|---|---|---|---|---|---|
| 1.41 | 3-F | $-CH=CH-CF_3$ | $CH_3$ | $OCH_3$ | H | N | |
| 1.42 | 3-F | $-CH=CH-CF_3$ | $OCH_3$ | $OCH_3$ | H | N | |
| 1.43 | H | $-CH=CH-C_6F_{13}$-n | $CH_3$ | $CH_3$ | H | CH | Smp. 134-138°C |
| 1.44 | H | $-CH=CH-C_6F_{13}$-n | $CH_3$ | $OCH_3$ | H | CH | Smp. 114-116°C |
| 1.45 | H | $-CH=CH-C_6F_{13}$-n | $OCH_3$ | $OCH_3$ | H | CH | |
| 1.46 | H | $-CH=CH-C_6F_{13}$-n | $CH_3$ | $OCH_3$ | H | N | Smp. 133-136°C |
| 1.47 | H | $-CH=CH-C_6F_{13}$-n | $OCH_3$ | $OCH_3$ | H | N | |
| 1.48 | H | $-CH=CH-C_3F_7$-n | $CH_3$ | $OCH_3$ | H | N | Smp. 128-130°C |
| 1.49 | H | $-CH=CH-CF_2-CH_3$ | $CH_3$ | $OCH_3$ | H | N | Smp. 162-163°C |
| 1.50 | H | $-CH=C(CF_3)-CH_2CF_3$ | $CH_3$ | $OCH_3$ | H | N | Smp. 164-165°C |
| 1.51 | H | $-CH=C(CH_3)-CF_3$ | $CH_3$ | $OCH_3$ | H | CH | Smp. 161-162°C |
| 1.52 | H | $-CH=CH-CF_3$ | $CH_3$ | $OC_2H_5$ | H | N | Smp. 146-149°C |
| 1.53 | H | $-CH=CHC_3F_7n$ | $OCH_3$ | $OCH_3$ | H | N | |
| 1.54 | H | $-CH=CHC_3F_7n$ | $CH_3$ | $OCH_3$ | H | CH | |
| 1.55 | H | $-CH=CHC_3F_7n$ | $OCH_3$ | $OCH_3$ | H | CH | |
| 1.56 | H | $-CH=CH-CF_2-CH_3$ | $OCH_3$ | $OCH_3$ | H | N | |
| 1.57 | H | $-CH=CH-CF_2-CH_3$ | $OCH_3$ | $CH_3$ | H | CH | |
| 1.58 | H | $-CH=CH-CF_2-CH_3$ | $OCH_3$ | $OCH_3$ | H | CH | |
| 1.59 | H | $-CH=CH-CF_3$ | $OCH_3$ | $OCHF_2$ | H | CH | |
| 1.60 | H | $-CH=CH-CF_3$ | $OCHF_2$ | $OCHF_2$ | H | CH | |
| 1.61 | H | $-CH=CH-CF_3$ | $OCH_3$ | $OCH_2CH_3$ | H | N | |
| 1.62 | H | $-CH=CH-CF_3$ | $OCH_2CH_3$ | $OCH_2CH_3$ | H | N | |
| 1.63 | H | $-CH=CH-CF_3$ | $CH_2CH_3$ | $OCH_3$ | H | N | |
| 1.64 | 5-$CH_3$ | $-CH=CH-CF_3$ | $CH_3$ | $OCH_3$ | H | N | Smp. 161-163°C |
| 1.65 | 5-$CH_3$ | $-CH=CH-CF_3$ | $OCH_3$ | $OCH_3$ | H | N | |
| 1.66 | 5-$CH_3$ | $-CH=CH-CF_3$ | $CH_3$ | $OCH_3$ | H | CH | |
| 1.67 | 5-$CH_3$ | $-CH=CH-CF_3$ | $OCH_3$ | $OCH_3$ | H | CH | |
| 1.68 | 5-$NO_2$ | $-CH=CH-CF_3$ | $CH_3$ | $OCH_3$ | H | N | Smp. 170-172°C |
| 1.69 | 5-$NO_2$ | $-CH=CH-CF_3$ | $OCH_3$ | $OCH_3$ | H | N | |
| 1.70 | 5-$NO_2$ | $-CH=CH-CF_3$ | $CH_3$ | $OCH_3$ | H | CH | |
| 1.71 | 5-$NO_2$ | $-CH=CH-CF_3$ | $OCH_3$ | $OCH_3$ | H | CH | |

**Tabelle 2:**

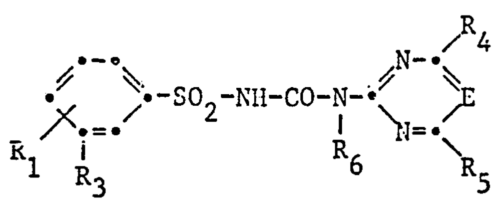

| Nr. | $R_1$ | $R_3$ | $R_4$ | $R_5$ | $R_6$ | E | phys.Daten |
|---|---|---|---|---|---|---|---|
| 2.1 | H | $-CH=CH-CF_3$ | $CH_3$ | $OCH_3$ | H | N | Smp. 161-162°C |
| 2.2 | H | $-CH=CH-CF_3$ | $CH_3$ | $OCH_3$ | H | CH | |
| 2.3 | H | $-CH=CH-CF_3$ | $CH_3$ | $OCH_3$ | H | CH | |
| 2.4 | H | $-CH=CH-CF_3$ | $OCH_3$ | $OCH_3$ | H | N | |

**Tabelle 3:**

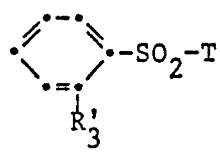

| Nr. | $R'_3$ | T | phys Daten |
|---|---|---|---|
| 3.1 | $-CH=CH-CF_3$ | $NH_2$ | Smp. 153-154°C |
| 3.2 | $-CH=CH-CF_3$ | $-NH-CO-NHCH_3$ | Smp. 190-192°C |
| 3.3 | $-CH=CH-CF_3$ | $-N=C=O$ | Öl |
| 3.4 | $-CH=CH-CF_3$ | $-NH-CO-OC_6H_5$ | |
| 3.5 | $-CH=CH-C_6F_{13}-n$ | $NH_2$ | Smp. 60-61°C |
| 3.6 | $-CH=CH-C_3F_7-n$ | $NH_2$ | Smp. 63-64°C |
| 3.7 | $-CH=CH-CF_2CH_3$ | $NH_2$ | Smp. 105-106°C |
| 3.8 | $-CH=C(CF_3)-CH_2-CF_3$ | $NH_2$ | |
| 3.9 | $-CH=C(CH_3)-CF_3$ | $NH_2$ | |
| 3.10 | $-CH=CH-CF_3$ | Cl | Smp. 43-44°C |
| 3.11 | $-CH=CH-C_3F_7-n$ | Cl | |
| 3.12 | $-CH=CF_2$ | Cl | |
| 3.13 | $-CH=CF_2$ | $NH_2$ | |
| 3.14 | $-CH=CF_2$ | $-N=C=O$ | |

**Tabelle 4:**

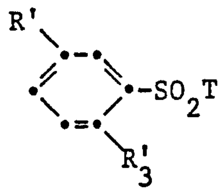

| Nr. | R' | $R'_3$ | T | phys. Daten |
|---|---|---|---|---|
| 4.1 | $CH_3$ | $-CH=CH-CF_3$ | $NH_2$ | Smp. 153-154°C |
| 4.2 | $NO_2$ | $-CH=CH-CF_3$ | $NH_2$ | Smp. 176-178°C |
| 4.3 | $C_2H_5$ | $-CH=CH-CF_3$ | $NH_2$ | |
| 4.4 | $C_2H_5$ | $-CH=CH-CF_3$ | $-N=C=O$ | |

**Formulierungsbeispiele**

**Beispiel 5:** Formulierungsbeispiele für Wirkstoffe der Formel I (% = Gewichtsprozent)

| a) Spritzpulver | a) | b) | c) |
|---|---|---|---|
| Wirkstoff | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther (7 - 8 Mol ÄO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) Emulsion-Konzentrat

| | a) | b) |
|---|---|---|
| Wirkstoff | 10 % | 1 % |
| Octylphenolpolyäthylenglykoläther (4 - 5 Mol ÄO) | 3 % | 3 % |
| Ca-Dodecylbenzolsulfonat | 3 % | 3 % |
| Ricinusölpolyglykoläther (36 Mol ÄO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

c) Stäubemittel

| | a) | b) |
|---|---|---|
| Wirkstoff | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

d) Extruder Granulat

| | a) | b) |
|---|---|---|
| Wirkstoff | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmäßig aufgetragen. Auf diese Weise erhält man staubfreie Umhüllungs-Granulate.

f) Suspensions-Konzentrat

| | a) | b) |
|---|---|---|
| Wirkstoff | 40 % | 5 % |
| Äthylenglykol | 10 % | 10 % |
| Nonylphenolpolyäthylenglykoläther (15 Mol ÄO) | 6 % | 1 % |
| Na-Ligninsulfonat | 10 % | 5 % |
| Carboxymethylcellulose | 1 % | 1 % |
| 37 %-ige wäßrige Formaldehyd-Lösung | 0,2 % | 0,2 % |
| Silikonöl in Form einer 75 %-igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff | 5 % |
| Isopropylamin | 1 % |
| Octylphenolpolyäthylenglykoläther (78 Mol ÄO) | 3 % |
| Wasser | 91 % |

**Biologische Beispiele**

**Beispiel 6:** Herbizidwirkung vor dem Auflaufen der Pflanzen

Kunststofftöpfe werden mit expandiertem Vermiculit (Dichte: 0,135 g/cm$^3$, Wasserabsorptionsvermögen: 0,565 1/1) gefüllt. Nach dem Sättigen des nicht adsorptiven Vermiculits mit einer wäßrigen Wirkstoffemulsion

in deionisiertem Wasser, die die Wirkstoffe in einer Konzentration von 70,8 ppm enthält, werden Samen der folgenden Pflanzen auf die Oberfläche gesät: Nasturtium officinalis, Agrostis tenuis, Stellaria media und Digitaria sanguinalis. Die Versuchsgefässe werden anschließend in einer Klimakammer bei 20°C, einer Beleuchtung von ca. 20 kLux und einer relativen Luftfeuchtigkeit von 70 % gehalten. Während der Keimphase von 4 bis 5 Tagen werden die Töpfe zur Erhöhung der örtlichen Luftfeuchtigkeit mit lichtdurchläßigem Material abgedeckt und mit deionisiertem Wasser gegossen. Nach dem 5. Tag wird dem Gießwasser 0,5 % eines handelsüblichen Flüssigdüngers (®Greenzit) zugesetzt. 12 Tage nach der Aussaat wird der Versuch ausgewertet und die Wirkung auf die Versuchspflanzen nach dem folgenden Maßstab bewertet:

1: Pflanze nicht gekennt oder total abgestorben
2-3: sehr starke Wirkung
4-6: mittlere Wirkung
7-8: schwache Wirkung
9: keine Wirkung (wie unbehandelte Kontrolle).

**pre-emergente Wirkung:**

Konzentration der Wirkstoffemulsion: 70,8 ppm

| Testpflanze<br>Wirkstoff Nr. | Nasturtium | Stellaria | Agrostis | Digitaria |
|---|---|---|---|---|
| 1.1 | 2 | 1 | 2 | 2 |
| 1.2 | 2 | 2 | 6 | 7 |
| 1.3 | 1 | 1 | 1 | 1 |
| 1.4 | 1 | 2 | 1 | 2 |
| 1.5 | 2 | 3 | 2 | 5 |
| 1.6 | 2 | 2 | 2 | 4 |
| 1.7 | 2 | 2 | 5 | 7 |
| 1.12 | 1 | 2 | 1 | 3 |
| 1.43 | 5 | 2 | 6 | 2 |
| 1.48 | 4 | 2 | 6 | 7 |
| 1.49 | 3 | 3 | 3 | 4 |
| 1.51 | 1 | 3 | 1 | 4 |
| 1.52 | 2 | 2 | 2 | 3 |
| 2.1 | 2 | 2 | 2 | 6 |

**Beispiel 7:** Nachweis der Selektivität bei Vorauflaufanwendung.

In der für pre-emergente Tests üblichen Versuchsanordnung werden eine größere Anzahl von Pflanzensamen mit verschiedenen Aufwandungmengen an Wirksubstanz behandelt. Die Auswertung erfolgt nach dem gleichen Maßstab wie im Beispiel 6.

**pre-emergente Wirkung:**

| Wirkung | Verb. Nr. 1.1 | |
|---|---|---|
| Aufwandmenge kg AS/ha | 0,125 | 0,06 |
| Testpflanze | | |
| Mais | 8 | 9 |
| Weizen | 9 | 9 |
| Alopecurus myos. | 3 | 4 |
| Cyperus escul. | 3 | 4 |
| Rottboellia ex. | 5 | 5 |
| Abutilon | 2 | 2 |
| Xanthium Sp. | 2 | 2 |
| Chenopodium Sp. | 2 | 2 |
| Ipomoea | 2 | 3 |
| Sinapis | 2 | 2 |
| Galium aparine | 2 | 2 |
| Viola tricolor | 2 | 2 |

**Beispiel 8:** <u>Nachweis der Herbizidwirkung nach dem Auflaufen der Pfanzen (Kontaktwirkung)</u>

Eine Anzahl Unkräuter und Kulturpflanzen, sowohl monokotyle wie dikotyle, wurden nach dem Auflaufen im 4- bis 6-Blattstadium mit einer wäßrigen Wirkstoffdispersion in Dosierungen von 0,5 kg AS/ha gespritzt und dann bei 24° bis 26°C und 45 - 60 % relativer Luftfeuchtigkeit gehalten. 15 Tage nach der Behandlung wird der Versuch ausgewertet und nach dem gleichen Maßstab wie im Beispiel 6 bewertet.

**post-emergente Wirkung**

Aufwandmenge: 0,5 kg Wirksubstanz/Hektar

| Verb. Nr. | Avena | Setaria | Lolium | Solanum | Sinapis | Stellaria | Phaseolus |
|---|---|---|---|---|---|---|---|
| 1.1 | 4 | 4 | 3 | 2 | 2 | 2 | 3 |

**Beispiel 9:** <u>Nachweis der Selektivität bei Nachauflaufanwendung</u>

In der gleichen Versuchsanordnung wie im Beispiel 8 werden eine größere Anzahl von Pflanzen mit verschiedenen Aufwandmengen an Wirksubstanz behandelt. Die Auswertung erfolgte nach dem in Beispiel 6 angegebenen Maßstab.

**pre-emergente Wirkung:**

| Wirkung | Verb. Nr. 1.1 | |
|---|---|---|
| Aufwandmenge kg AS/ha | 0,125 | 0,125 |
| Testpflanze | | |
| Weizen | 9 | 9 |
| Mais | 7 | 9 |
| Reis trocken | 8 | 8 |
| Cyperus escul. | 3 | 4 |
| Abutilon | 3 | 4 |
| Xanthium Sp. | 3 | 4 |
| Chenopodium Sp. | 2 | 2 |
| Sinapis | 3 | 3 |
| Galium aparine | 3 | 4 |
| Viola tricolor | 2 | 3 |

**Beispiel 10:** <u>Nachweis der Wuchshemmung bei tropischen Bodendeckern-Leguminosen (cover crops).</u>

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff

als wäßrige Emulsion gespritzt. Die Versuchspflanzen werden bei 70 % relativer Luftfeuchtigkeit und 6000 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20 % des Neuzuwachses bei unbehandelten Kontrollpflanzen) ohne daß dabei die Versuchspflanzen geschädigt wurden.

**Beispiel 11:** Wuchsregulierung an Sojabohnen

In Kunststoffbehältern mit einem Erde-Torf-Sandgemisch im Verhältnis 6 : 3 : 1 werden Sojabohnen der Sorte "Hark" angesät und in eine Klimakammer gegeben. Durch optimale Temperaturwahl, Beleuchtung, Düngerzugabe und Bewäßerung entwickeln sich die Pflanzen nach ca. 5 Wochen bis zum 5 - 6 Trifolia-Blattstadium. Zu diesem Zeitpunkt werden die Pflanzen mit der wäßrigen Brühe eines Wirkstoffes der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemäßen Wirkstoffe der Formel I eine merkliche Erhöhung der Anzahl und des Gewichts der Schoten am Haupttrieb.

**Beispiel 12:** Wuchshemmung bei Getreide

In Kunststofftöpfen mit sterilisierter Erde werden die Getreidearten Hordeum vulgare (Sommergerste) und Secale (Sommerroggen) im Gewächshaus angesät und nach Bedarf bewässert. Die Sprößlinge werden ca. 21 Tage nach der Aussaat mit der wäßrigen Spritzbrühe eines Wirkstoffes der Formel I besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zur unbehandelten Kontrolle eine Verringerung des Neuzuwachses (60 - 90 % der Kontrolle), sowie teilweise eine Zunahme der Stengeldurchmesser auf.

**Beispiel 13:** Wuchshemmung bei Gräsern

In Kunststoffschalen mit Erde-Torf-Sand-Gemisch (6 : 3 : 1) werden die Gräser Lolium perenne, Poa pratensis, Festuca ovina, Dactylis glomerata und Cynodon dactylon im Gewächshaus angesät und nach Bedarf bewässert. Die aufgelaufenen Gräser werden wöchtentlich bis auf 4 cm Höhe zurückgeschnitten und ca. 50 Tage nach der Aussaat und einen Tag nach dem letzten Schnitt mit der wäßrigen Spritzbrühe eines Wirkstoffes der Formel I besprüht. Die Wirkstoffmenge beträgt umgerechnet bis zu 100 g Aktivsubstanz pro Hektar. 21 Tage nach Applikation wird das Wachstum der Gräser beurteilt.

Die Verbindungen der Formel I bewirken eine Reduzierung des Neuzuwachses von um 10 - 30 % im Vergleich zur unbehandelten Kontrolle.

**Patentansprüche** für die Vertragsstaaten: GE, BE, DE, FR, IT, NL, SE, CH

1. N-phenylsulfonyl-N'-pyrimidinyl- und -triazinyl-harnstoffe der Formel I

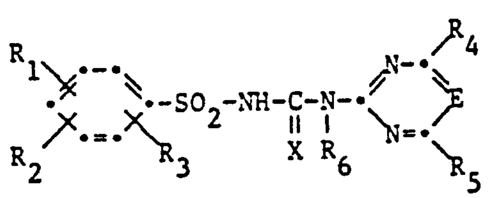

(I),

worin

$R_1$ Wasserstoff, Halogen, Nitro, Amino, $C_1$-$C_5$-Alkyl, $C_1$-$C_4$-Halogenalkyl oder einen Rest -Q-$R_7$, -CO-$OR_8$ oder -$(CO)_n$-$NR_9R_{10}$,

$R_2$ Wasserstoff, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Halogen oder Alkoxyalkyl mit höchstens 4 Kohlenstoffatomen,

$R_3$ einfach oder mehrfach durch Fluor Substituiertes $C_2$-$C_{10}$-Alkenyl, dessen Doppelbindung direkt an den Phenylkern gebunden ist,

$R_4$ $C_1$-$C_3$-Alkyl, $C_1$-$C_3$-Halogenalkyl, $C_1$-$C_3$-Alkoxy oder $C_1$-$C_3$-Halogenalkoxy,

$R_5$ Wasserstoff, Halogen, -$NR_{13}R_{14}$, $C_1$-$C_3$-Alkyl, $C_1$-$C_3$-Halogenalkyl, $C_1$-$C_3$-Alkoxy oder $C_1$-$C_2$-Halogenalkoxy,

$R_6$ Wasserstoff, $C_1$-$C_3$-Alkyl oder $C_1$-$C_3$-Alkoxy,

X Sauerstoff oder Schwefel und

E Stickstoff oder die Methingruppe bedeuten, wobei
$R_7$ für gegebenenfalls durch Halogen oder $C_1$-$C_3$-Alkoxy substituiertes $C_1$-$C_4$-Alkyl oder $C_3$-$C_5$-Alkenyl,
$R_8$ für $C_1$-$C_4$-Alkyl, $C_1$-$C_4$-Halogenalkyl oder $C_2$-$C_6$-Alkoxyalkyl,
$R_9$, $R_{10}$, $R_{13}$ und $R_{14}$ unabhängig voneinander für Wasserstoff oder $C_1$-$C_3$-Alkyl,
Q für Sauerstoff, Schwefel, die Sulfinyl- oder Sulfonylbrücke, und
n für Null oder eins stehen,
sowie die Salze dieser Verbindungen.

2. Verbindungen gemäß Anspruch 1, dadurch gekennzeichnet, daß X Sauerstoff bedeutet.

3. Verbindungen gemäß Anspruch 1, dadurch gekennzeichnet, daß $R_4$ und $R_5$ zusammen nicht mehr als 4 Kohlenstoffatome enthalten.

4. Verbindungen gemäß Anspruch 1, dadurch gekennzeichnet, daß $R_6$ Wasserstoff bedeutet.

5. Verbindungen gemäß Anspruch 1, dadurch gekennzeichnet, daß $R_1$ und $R_2$ Wasserstoff bedeuten.

6. Verbindungen gemäß Anspruch 1, dadurch gekennzeichnet, daß $R_3$ die 2-Stellung zur Sulfonylgruppe besetzt.

7. Verbindungen gemäß Anspruch 1, dadurch gekennzeichnet, daß der Substituent $R_3$ 2 bis 4 Kohlenstoffatome enthält.

8. Verbindungen gemäß Anspruch 1, dadurch gekennzeichnet, daß X für Sauerstoff und $R_1$, $R_2$ und $R_6$ für Wasserstoff stehen, $R_4$ und $R_5$ zusammen nicht mehr als 4 Kohlenstoffatome enthalten und $R_3$ die 2-Stellung zur Sulfonylgruppe besetzt und 2 bis 4 Kohlenstoffatome enthält.

9. Verbindungen gemäß Anspruch 1, ausgewählt aus der Gruppe
N-[2-(3,3,3-Trifluor-1-propen-1-yl)-phenyl-sulfonyl]-N'-(4-methoxy-6-methyl-1,3,5-triazin-2-yl)-harnstoff oder
N-[2-(3,3,3-Trifluor-1-propen-1-yl)-phenyl-sulfonyl]-N'-(4,6-dimethoxy-1,3,5-triazin-2-yl)-harnstoff.

10. Verfahren zur Herstellung der Verbindungen der Formel I, Anspruch 1, dadurch gekennzeichnet, daß man ein Phenylsulfonamid der Formel II

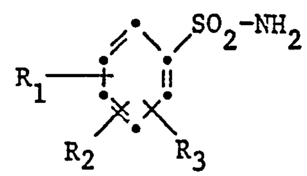
(II),

worin $R_1$, $R_2$ und $R_3$ die unter Formel I im Anspruch 1 gegebene Bedeutung haben, in Gegenwart einer Base mit einem N-Pyrimidinyl- oder -Triazinylcarbamat der Formel III

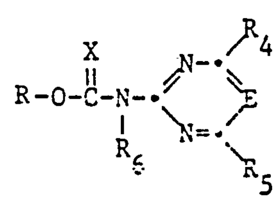
(III),

worin E, $R_4$, $R_5$, $R_6$ und X die unter Formel I im Anspruch 1 gegebene Bedeutung haben und R für Phenyl, Alkyl oder substituiertes Phenyl steht, umsetzt und gegebenenfalls in ihre Salze überführt.

11. Verfahren zur Herstellung der Verbindungen der Formel I, Anspruch 1, dadurch gekennzeichnet, daß man ein phenylsulfonylisocyanat oder -isothiocyanat der Formel IV

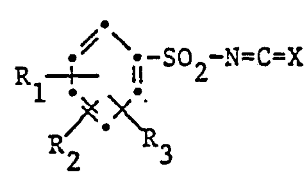
(IV),

worin $R_1$, $R_2$, $R_3$ und X die unter Formel I im Anspruch 1 gegebene Bedeutung haben, gegebenenfalls in Gegenwart einer Base, mit einem Amin der Formel V

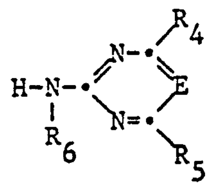

(V),

worin E, $R_4$, $R_5$ und $R_6$ die unter Formel I im Anspruch 1 gegebene Bedeutung haben, umsetzt und gegebenenfalls in ihre Salze überführt.

12. Verfahren zur Herstellung der Verbindungen der Formel I, Anspruch 1, dadurch gekennzeichnet, daß man Sulfonamide der oben angegebenen Formel II gegebenenfalls in Gegenwart einer Base mit einem Isocyanat oder Isothiocyanat der Formel VI

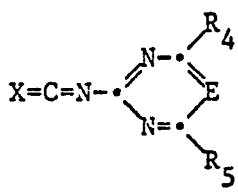

(VI),

worin E, $R_4$, $R_5$ und X die unter Formel I im Anspruch 1 gegebene Bedeutung haben, umsetzt und gegebenenfalls in ihre Salze überführt.

13. Verfahren zur Herstellung der Verbindungen der Formel I, Anspruch 1, dadurch gekennzeichnet, daß man ein N-Phenylsulfonylcarbamat der Formel VII

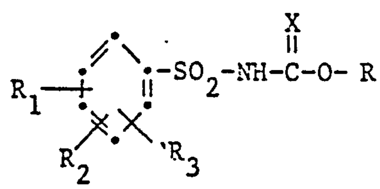

(VII),

worin $R_1$, $R_2$, $R_3$ und X die unter Formel I im Anspruch 1 gegebene Bedeutung haben und R für Phenyl, Alkyl oder substituiertes Phenyl steht, mit einem Amin der im Anspruch 11 oben angegebenen Formel V umsetzt und gegebenenfalls in ihre Salze überführt.

14. Verfahren zur Herstellung von Additionssalzen der Formel I gemäß einem der Ansprüche 10 bis 13, dadurch gekennzeichnet, daß man einen Sulfonylharnstoff der Formel I mit einem Amin, einem Alkalimetall- oder Erdalkalimetallhydroxid oder einer quaternären Ammoniumbase umsetzt.

15. Ein herbizides und den Pflanzenwuchs regulierendes Mittel, dadurch gekennzeichnet, daß es neben Träger- und/oder anderen Zuschlagstoffen als Wirkstoff mindestens einen N-Phenylsulfonyl-N'-triazinyl- oder -pyrimidinyl-harnstoff der Formel I, Anspruch 1, enthält.

16. Die Verwendung der N-Phenylsulfonyl-N'-triazinyl- oder -pyrimidinyl-harnstoffe der Formel I, Anspruch 1, oder sie enthaltender Mittel zur Bekämpfung unerwünschten Pflanzenwachstums.

17. Die Verwendung der N-Phenylsulfonyl-N'-triazinyl- oder -pyrimidinyl-harnstoffe der Formel I, Anspruch 1, oder sie enthaltender Mittel zur Regulierung des Pflanzenwachstums.

18. Die Verwendung der N-Phenylsulfonyl-N'-triazinyl- oder -pyrimidinyl-harnstoffe der Formel I, Anspruch 1, oder sie enthaltender Mittel zur Wuchsregulierung von Kulturpflanzen zum Zwecke einer Ertragssteigerung.

19. Die Verwendung der N-Phenylsulfonyl-N'-triazinyl- oder -pyrimidinyl-harnstoffe der Formel I, oder sie enthaltender Mittel, gemäß Anspruch 16, zur selektiven pre- oder postemergenten Bekämpfung von Unkräutern in Nutzpflanzenkulturen.

20. Die Verwendung der Verbindungen der Formel I oder sie enthaltender Mittel gemäß Anspruch 17 im Getreide, Mais und Reis.

21. Die Verwendung der Verbindungen der Formel I oder sie enthaltender Mittel gemäß Anspruch 18 in Soja.

**Patentansprüche** für den Vertragsstaat: AT

1. Ein herbizides und den Pflanzenwuchs regulierendes Mittel, dadurch gekennzeichnet, daß es neben 1 - 99,9 % Träger- und/oder anderen Zuschlagstoffen als Wirkstoff mindestens einen N-Phenylsulfonyl-N'-triazinyl oder -pyrimidinyl-harnstoff der Formel I

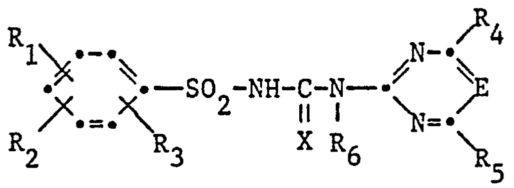

(I),

oder ein Salz davon in einer Konzentration von 0,1 - 95 % enthält, worin

$R_1$ Wasserstoff, Halogen, Nitro, Amino, $C_1$-$C_5$-Alkyl, $C_1$-$C_4$-Halogenalkyl oder einen Rest -Q-$R_7$, -CO-$OR_8$ oder -$(CO)_n$-$NR_9R_{10}$,

$R_2$ Wasserstoff, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Halogen oder Alkoxyalkyl mit höchstens 4 Kohlenstoffatomen,

$R_3$ einfach oder mehrfach durch Fluor Substituiertes $C_2$-$C_{10}$-Alkenyl, dessen Doppelbindung direkt an den Phenylkern gebunden ist,

$R_4$ $C_1$-$C_3$-Alkyl, $C_1$-$C_3$-Halogenalkyl, $C_1$-$C_3$-Alkoxy oder $C_1$-$C_3$-Halogenalkoxy,

$R_5$ Wasserstoff, Halogen, -$NR_{13}R_{14}$, $C_1$-$C_3$-Alkyl, $C_1$-$C_3$-Halogenalkyl, $C_1$-$C_3$-Alkoxy oder $C_1$-$C_2$-Halogenalkoxy,

$R_6$ Wasserstoff, $C_1$-$C_3$-Alkyl oder $C_1$-$C_3$-Alkoxy,

X Sauerstoff oder Schwefel und

E Stickstoff oder die Methingruppe bedeuten, wobei

$R_7$ für gegebenenfalls durch Halogen oder $C_1$-$C_3$-Alkoxy substituiertes $C_1$-$C_4$-Alkyl oder $C_3$-$C_5$-Alkenyl,

$R_8$ für $C_1$-$C_4$-Alkyl, $C_1$-$C_4$-Halogenalkyl oder $C_2$-$C_6$-Alkoxyalkyl,

$R_9$, $R_{10}$, $R_{13}$ und $R_{14}$ unabhängig voneinander für Wasserstoff oder $C_1$-$C_3$-Alkyl,

Q für Sauerstoff, Schwefel, die Sulfinyl- oder Sulfonylbrücke, und

n für Null oder eins stehen.

2. Mittel gemäß Anspruch 1, dadurch gekennzeichnet, daß X Sauerstoff bedeutet.

3. Mittel gemäß Anspruch 1, dadurch gekennzeichnet, daß $R_4$ und $R_5$ zusammen nicht mehr als 4 Kohlenstoffatome enthalten.

4. Mittel gemäß Anspruch 1, dadurch gekennzeichnet, daß $R_6$ Wasserstoff bedeutet.

5. Mittel gemäß Anspruch 1, dadurch gekennzeichnet, daß $R_1$ und $R_2$ Wasserstoff bedeuten.

6. Mittel gemäß Anspruch 1, dadurch gekennzeichnet, daß $R_3$ die 2-Stellung zur Sulfonylgruppe besetzt.

7. Mittel gemäß Anspruch 1, dadurch gekennzeichnet, daß der Substituent $R_3$ 2 bis 4 Kohlenstoffatome enthält.

8. Mittel gemäß Anspruch 1, dadurch gekennzeichnet, daß X für Sauerstoff und $R_1$, $R_2$ und $R_6$ für Wasserstoff stehen, $R_4$ und $R_5$ zusammen nicht mehr als 4 Kohlenstoffatome enthalten und $R_3$ die 2-Stellung zur Sulfonylgruppe besetzt und 2 bis 4 Kohlenstoffatome enthält.

9. Mittel gemäß Anspruch 1 mit einem Wirkstoff, ausgewählt aus der Gruppe
N-[2-(3,3,3-Trifluor-1-propen-1-yl)-phenyl-sulfonyl]-N'-(4-methoxy-6-methyl-1,3,5-triazin-2-yl)-harnstoff oder
N-[2-(3,3,3-Trifluor-1-propen-1-yl)-phenyl-sulfonyl]-N'-(4,6-dimethoxy-1,3,5-triazin-2-yl)-harnstoff.

10. Verfahren zur Herstellung der Verbindungen der Formel I, Anspruch 1, dadurch gekennzeichnet, daß man entweder ein Phenylsulfonamid der Formel II

a)

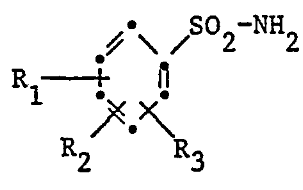

(II),

worin $R_1$, $R_2$ und $R_3$ die unter Formel I im Anspruch 1 gegebene Bedeutung haben, in Gegenwart einer Base mit einem N-Pyrimidinyl- oder -Triazinylcarbamat der Formel III

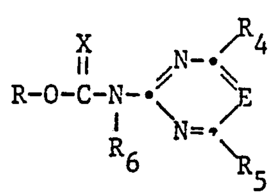

(III),

worin E, $R_4$, $R_5$, $R_6$ und X die unter Formel I im Anspruch 1 gegebene Bedeutung haben und R für phenyl, Alkyl oder substituiertes Phenyl steht, umsetzt oder

b) ein Phenylsulfonylisocyanat oder -isothiocyanat der Formel IV

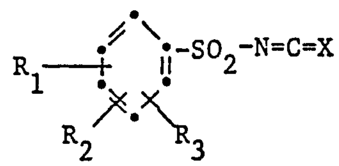

(IV),

worin $R_1$, $R_2$, $R_3$ und X die unter Formel I im Anspruch 1 gegebene Bedeutung haben, gegebenenfalls in Gegenwart einer Base, mit einem Amin der Formel V

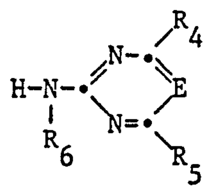

(V),

worin E, $R_4$, $R_5$ und $R_6$ die unter Formel I im Anspruch 1 gegebene Bedeutung haben, oder

c) man Sulfonamide der oben angegebenen Formel II gegebenenfalls in Gegenwart einer Base mit einem Isocyanat oder Isothiocyanat der Formel VI

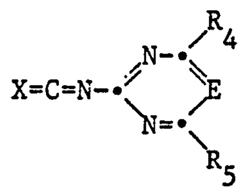

(VI),

worin E, $R_4$, $R_5$ und X die unter Formel I im Anspruch 1 gegebene Bedeutung haben, umsetzt, oder

d) ein N-Phenylsulfonylcarbamat der Formel VII

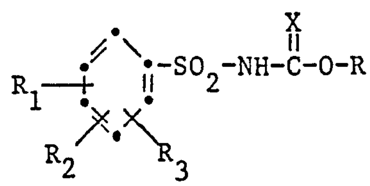

(VII),

worin $R_1$, $R_2$, $R_3$ und X die unter Formel I im Anspruch 1 gegebene Bedeutung haben und R für Phenyl, Alkyl oder substituiertes Phenyl steht, mit einem Amin der oben angegebenen Formel V umsetzt und gegebenenfalls in ihre Salze überführt, indem man einen Sulfonylharnstoff der Formel I mit einem Amin, einem Alkalimetall- oder Erdalkalimetallhydroxid oder einer quaternären Ammoniumbase umsetzt.

11. Die Verwendung der N-Phenylsulfonyl-N'-triazinyl- oder -pyrimidinyl-harnstoffe der Formel I, Anspruch 1, oder sie enthaltender Mittel zur Bekämpfung unerwünschten Pflanzenwachstums.

12. Die Verwendung der N-Phenylsulfonyl-N'-triazinyl- oder -pyrimidinyl-harnstoffe der Formel I, Anspruch 1, oder sie enthaltender Mittel zur Regulierung des Pflanzenwachstums.

13. Die Verwendung der N-Phenylsulfonyl-N'-triazinyl- oder -pyrimidinyl-harnstoffe der Formel I, Anspruch 1, oder sie enthaltender Mittel zur Wuchsregulierung von Kulturpflanzen zum Zwecke einer Ertragssteigerung.

14. Die Verwendung der N-Phenylsulfonyl-N'-triazinyl- oder -pyrimidinyl-harnstoffe der Formel I, oder sie enthaltender Mittel, gemäß Anspruch 11, zur selektiven pre- oder postemergenten Bekämpfung von Unkräutern in Nutzpflanzenkulturen.

15. Die Verwendung der Verbindungen der Formel I oder sie enthaltende Mittel gemäß Anspruch 12 im Getreide, Mais und Reis.

16. Die Verwendung der Verbindungen der Formel I oder sie enthaltende Mittel gemäß Anspruch 13 in Soja.

**Claims** for the Contracting States: GB, BE, DE, FR, IT, NL, SE, CH

1. N-phenylsulfonyl-N'-pyrimidinylureas and N-phenylsulfonyl-N'-triazinylureas of the formula I

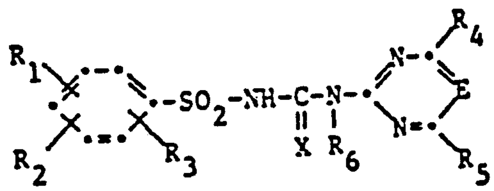

(I),

wherein

$R_1$ is hydrogen, halogen, nitro, amino, $C_1$-$C_5$alkyl, $C_1$-$C_4$haloalkyl or a -Q-$R_7$, -CO-$OR_8$ or -$(CO)_n$-$NR_9R_{10}$ radical,

$R_2$ is hydrogen, $C_1$-$C_4$alkyl, $C_1$-$C_4$alkoxy, $C_1$-$C_4$alkylthio, $C_1$-$C_4$haloalkyl, $C_1$-$C_4$haloalkoxy, halogen or alkoxyalkyl containing not more than 4 carbon atoms,

$R_3$ is $C_2$-$C_{10}$alkenyl which is substituted by one or more fluorine atoms and the double bond of which is directly bonded to the phenyl nucleus,

$R_4$ is $C_1$-$C_3$alkyl, $C_1$-$C_3$haloalkyl, $C_1$-$C_3$alkoxy or $C_1$-$C_3$haloalkoxy,

$R_5$ is hydrogen, halogen, -$NR_{13}R_{14}$, $C_1$-$C_3$alkyl, $C_1$-$C_3$haloalkyl, $C_1$-$C_3$alkoxy or $C_1$-$C_2$haloalkoxy,

$R_6$ is hydrogen, $C_1$-$C_3$alkyl or $C_1$-$C_3$alkoxy,

X is oxygen or sulfur, and

E is nitrogen or the methine group, and

$R_7$ is $C_1$-$C_4$alkyl which is unsubstituted or substituted by halogen or $C_1$-$C_3$alkoxy or is $C_3$-$C_5$alkenyl,

$R_8$ is $C_1$-$C_4$alkyl, $C_1$-$C_4$haloalkyl or $C_2$-$C_6$alkoxyalkyl,

$R_9$, $R_{10}$, $R_{13}$ and $R_{14}$, each independently of the others, are hydrogen or $C_1$-$C_3$alkyl,

Q is oxygen, sulfur, the sulfinyl or sulfonyl bridge, and

n is 0 or 1,

or a salt thereof.

2. Compounds according to claim 1, characterised in that X is oxygen.

3. Compounds according to claim 1, characterised in that $R_4$ and $R_5$ together contain not more than 4 carbon atoms.

4. Compounds according to claim 1, characterised in that $R_6$ is hydrogen.

5. Compounds according to claim 1, characterised in that $R_1$ and $R_2$ are hydrogen.

6. Compounds according to claim 1, characterised in that $R_3$ is in the 2-position to the sulfonyl group.

7. Compounds according to claim 1, characterised in that the substituent $R_3$ contains 2 to 4 carbon atoms.

8. Compounds according to claim 1, characterised in that X is oxygen and $R_1$, $R_2$ and $R_6$ are hydrogen, $R_4$ and $R_5$ together contain not more than 4 carbon atoms and $R_3$ is in the 2-position to the sulfonyl group and contains 2 to 4 carbon atoms.

9. Compounds according to claim 1 selected from the group consisting of
N-[2-(3,3,3-trifluoro-1-propen-1-yl)-phenylsulfonyl]-N'-(4-methoxy-6-methyl-1,3,5-triazin-2-yl)urea,
or
N-[2-(3,3,3-trifluoro-1-propen-1-yl)phenylsulfonyl]-N'-(4,6-dimethoxy-1,3,5-triazin-2-yl)urea.

10. A process for the preparation of the compounds of the formula I, claim 1, characterised in that a phenylsulfonamide of the formula II

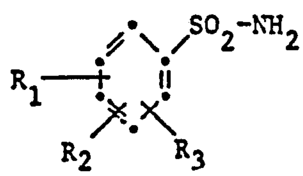

(II),

wherein $R_1$, $R_2$ and $R_3$ are as defined for formula I in claim 1, is reacted with an N-pyrimidinylcarbamate or N-triazinylcarbamate of the formula III

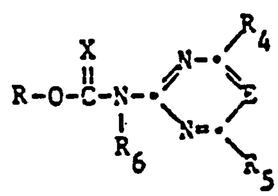

(III),

wherein E, $R_4$, $R_5$, $R_6$ and X are as defined for formula I in claim 1 and R is phenyl, alkyl or substituted phenyl, in the presence of a base, and, if desired, the compound so obtained is converted into a salt thereof.

11. A process for the preparation of the compounds of the formula I, claim 1, characterised in that a phenylsulfonyl isocyanate or isothiocyanate of the formula IV

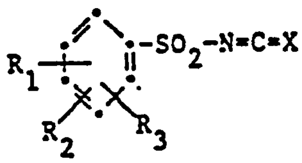

(IV),

wherein $R_1$, $R_2$, $R_3$ and X are as defined for formula I in claim 1, is reacted with an amine of the formula V

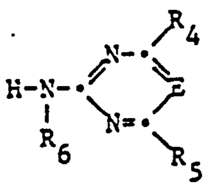

(V),

wherein E, $R_4$, $R_5$ and $R_6$ are as defined for formula I in claim 1, optionally in the presence of a base, and, if desired, the compound so obtained is converted into a salt thereof.

12. A process for the preparation of the compounds of the formula I, claim 1, characterised in that sulfonamides of the formula II above are reacted with an isocyanate or isothiocyanate of the formula VI

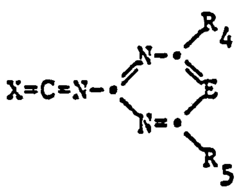

(VI),

wherein E, $R_4$, $R_5$ and X are as defined for formula I in claim 1, optionally in the presence of a base, and, if desired, the compound so obtained is converted into a salt thereof.

13. A process for the preparation of the compounds of the formula I, claim 1, characterised in that an N-phenylsulfonylcarbamate of the formula VII

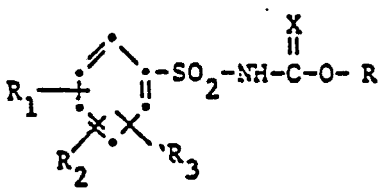

(VII),

wherein $R_1$, $R_2$, $R_3$ and X are as defined for formula I in claim 1 and R is phenyl, alkyl or substituted phenyl, is reacted with an amine of the formula V given in claim 11, and, if desired, the compound so obtained is converted into a salt thereof.

14. A process for the preparation of addition salts of the formula I according to any one of claims 10 to 13, characterised in that a sulfonylurea of the formula I is reacted with an amine, an alkali metal hydroxide or alkaline earth metal hydroxide or with a quaternary ammonium base.

15. A herbicidal and plant growth regulating composition, characterised in that it contains as active ingredient at least one N-phenylsulfonyl-N'-triazinylurea or N-phenylsulfonyl-N'-pyrimidinylurea of the formula I, claim 1, together with carriers and/or other adjuvants.

16. The use of the N-phenylsulfonyl-N'-triazinylureas or N-phenylsulfonyl-N'-pyrimidinylureas of the formula I, claim 1, or of compositions containing such compounds, for controlling undesired plant growth.

17. The use of the N-phenylsulfonyl-N'-triazinylureas or N-phenylsulfonyl-N'-pyrimidinylureas of the formula I, claim 1, or of compositions containing such compounds, for regulating plant growth.

18. The use of the N-phenylsulfonyl-N'-triazinylureas or N-phenylsulfonyl-N'-pyrimidinylureas of the formula I, claim 1, or of compositions containing such compounds, for regulating the growth of cultivated plants to increase yield.

19. The use of the N-phenylsulfonyl-N'-triazinylureas or N-phenylsulfonyl-N'-pyrimidinylureas of the formula I, or of compositions containing such compounds, according to claim 16, for selectively controlling weeds pre- or post-emergence in crops of useful plants.

20. The use of the compounds of the formula I, or of compositions containing such compounds, according to claim 17 in cereals, maize and rice.

21. The use of the compounds of the formula I, or of compositions containing such compounds, according to claim 18 in soybeans.

**Claims** for the Contracting State: AT

1. A herbicidal and plant growth regulating composition, characterised in that it contains as active ingredient, together with 1 to 99.9 % carriers and/or other adjuvants, at least one N-phenylsulfonyl-N'-triazinylurea or N-phenylsulfonyl-N'-pyrimidinylurea of the formula I

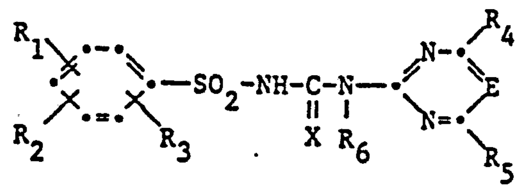

(I),

or a salt thereof, in a concentration of 0.1 to 95 %, wherein

$R_1$ is hydrogen, halogen, nitro, amino, $C_1$-$C_5$alkyl, $C_1$-$C_4$haloalkyl or a -Q-$R_7$, -CO-$OR_8$ or $-(CO)_n-NR_9R_{10}$ radical,
$R_2$ is hydrogen, $C_1$-$C_4$alkyl, $C_1$-$C_4$alkoxy, $C_1$-$C_4$alkylthio, $C_1$-$C_4$haloalkyl, $C_1$-$C_4$haloalkoxy, halogen or alkoxyalkyl containing not more than 4 carbon atoms,
$R_3$ is $C_2$-$C_{10}$alkenyl which is substituted by one or more fluorine atoms and the double bond of which is directly bonded to the phenyl nucleus,
$R_4$ is $C_1$-$C_3$alkyl, $C_1$-$C_3$haloalkyl, $C_1$-$C_3$alkoxy or $C_1$-$C_3$haloalkoxy,
$R_5$ is hydrogen, halogen, $-NR_{13}R_{14}$, $C_1$-$C_3$alkyl, $C_1$-$C_3$haloalkyl, $C_1$-$C_3$alkoxy or $C_1$-$C_2$haloalkoxy,
$R_6$ is hydrogen, $C_1$-$C_3$alkyl or $C_1$-$C_3$alkoxy,
X is oxygen or sulfur, and
E is nitrogen or the methine group, and
$R_7$ is $C_1$-$C_4$alkyl which is unsubstituted or substituted by halogen or $C_1$-$C_3$alkoxy or is $C_3$-$C_5$alkenyl,
$R_8$ is $C_1$-$C_4$alkyl, $C_1$-$C_4$haloalkyl or $C_2$-$C_6$alkoxyalkyl,
$R_9$, $R_{10}$, $R_{13}$ and $R_{14}$, each independently of the others, are hydrogen or $C_1$-$C_3$alkyl,
Q is oxygen, sulfur, the sulfinyl or sulfonyl bridge, and
n is 0 or 1.

2. A composition according to claim 1, characterised in that X is oxygen.

3. A composition according to claim 1, characterised in that $R_4$ and $R_5$ together contain not more than 4 carbon atoms.

4. A composition according to claim 1, characterised in that $R_6$ is hydrogen.

5. A composition according to claim 1, characterised in that $R_1$ and $R_2$ are hydrogen.

6. A composition according to claim 1, characterised in that $R_3$ is in the 2-position to the sulfonyl group.

7. A composition according to claim 1, characterised in that the substituent $R_3$ contains 2 to 4 carbon atoms.

8. A composition according to claim 1, characterised in that X is oxygen and $R_1$, $R_2$ and $R_6$ are hydrogen, $R_4$ and $R_5$ together contain not more than 4 carbon atoms and $R_3$ is in the 2-position to the sulfonyl group and contains 2 to 4 carbon atoms.

9. A composition according to claim 1 containing an active ingredient selected from the group consisting of
N-[2-(3,3,3-trifluoro-1-propen-1-yl)-phenylsulfonyl]-N'-(4-methoxy-6-methyl-1,3,5-triazin-2-yl)urea, or
N-[2-(3,3,3-trifluoro-1-propen-1-yl)phenylsulfonyl]-N'-(4,6-dimethoxy-1,3,5-triazin-2-yl)urea.

10. A process for the preparation of the compounds of the formula I, claim 1, characterised in that either

a) a phenylsulfonamide of the formula II

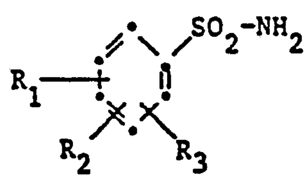

(II),

wherein $R_1$, $R_2$ and $R_3$ are as defined for formula I in claim 1, is reacted with an N-pyrimidinylcarbamate or N-triazinylcarbamate of the formula III

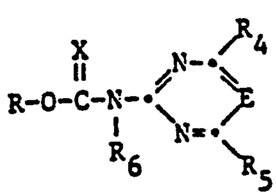

(III),

wherein E, $R_4$, $R_5$, $R_6$ and X are as defined for formula I in claim 1 and R is phenyl, alkyl or substituted phenyl, in the presence of a base,

or b) a phenylsulfonyl isocyanate or isothiocyanate of the formula IV

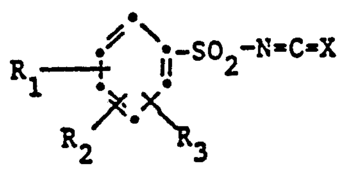

(IV),

wherein $R_1$, $R_2$, $R_3$ and X are as defined for formula I in claim 1, is reacted with an amine of the formula V

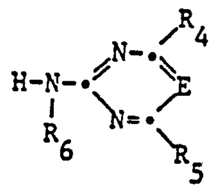

(V),

wherein E, $R_4$, $R_5$ and $R_6$ are as defined for formula I in claim 1, optionally in the presence of a base, or

c) sulfonamides of the formula II above are reacted with an isocyanate or isothiocyanate of the formula VI

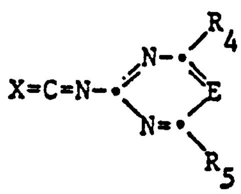

(VI),

wherein E, $R_4$, $R_5$ and X are as defined for formula I in claim 1, optionally in the presence of a base, or

d) an N-phenylsulfonylcarbamate of the formula VII

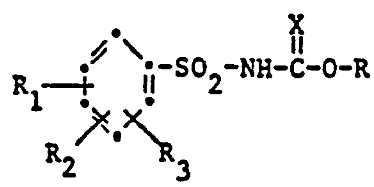

(VII),

wherein $R_1$, $R_2$, $R_3$ and X are as defined for formula I in claim 1 and R is phenyl, alkyl or substituted phenyl, is reacted with an amine of the formula V given above, and, if desired, the compound so obtained is converted into a salt thereof by reacting a sulfonylurea of the formula I with an amine, an alkali metal hydroxide or alkaline earth metal hydroxide or with a quaternary ammonium base.

11. The use of the N-phenylsulfonyl-N'-triazinylureas or N-phenylsulfonyl-N'-pyrimidinylureas of the formula I, claim 1, or of compositions containing such compounds, for controlling undesired plant growth.

12. The use of the N-phenylsulfonyl-N'-triazinylureas or N-phenylsulfonyl-N'-pyrimidinylureas of the formula I, claim 1, or of compositions containing such compounds, for regulating plant growth.

13. The use of the N-phenylsulfonyl-N'-triazinylureas or N-phenylsulfonyl-N'-pyrimidinylureas of the formula I, claim 1, or of compositions containing such compounds, for regulating the growth of cultivated plants to increase yield.

14. The use of the N-phenylsulfonyl-N'-triazinylureas or N-phenylsulfonyl-N'-pyrimidinylureas of the formula I, or of compositions containing such compounds, according to claim 11, for selectively controlling weeds pre- or post-emergence in crops of useful plants.

15. The use of the compounds of formula I or of compositions containing such compounds according to claim 12 in cereals, maize and rice.

16. The use of the compounds of the formula I or of compositions containing such compounds according to claim 13 in soybeans.

**Revendications** pour les Etats Contractants: GB, SE, DE, FR, IT, NL, SE, CH.

1. N-phénylsulfonyl-N'-pyrimidinyl- et -triazinyl-urées de formule I

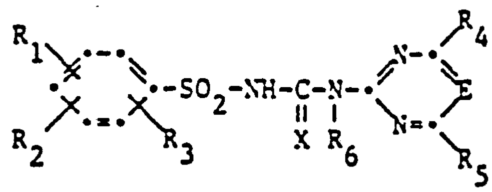

(I),

dans laquelle

$R_1$ represente l'hydrogène, un halogène, un groupe nitro, amino, alkyle en $C_1$-$C_5$, halogénoalkyle en $C_1$-$C_4$ ou un groupe -Q-$R_7$, -CO-O$R_8$ ou -(CO)$_n$-N$R_9R_{10}$,

$R_2$ représente l'hydrogène, un groupe alkyle en $C_1$-$C_4$, alcoxy en $C_1$-$C_4$, alkylthio en $C_1$-$C_4$, halogénoalkyle en $C_1$-$C_4$, halogénoalcoxy en $C_1$-$C_4$, un halogène ou un groupe alcoxyalkyle contenant au maximum 4 atomes de carbone,

$R_3$ représente un groupe alcényle en $C_2$-$C_{10}$ mono- ou poly-substitué par le fluor et dont la double liaison est reliée directement au noyau phényle,

$R_4$ représente un groupe alkyle en $C_1$-$C_3$, halogénoalkyle en $C_1$-$C_3$, alcoxy en $C_1$-$C_3$ ou halogénoalcoxy en $C_1$-$C_3$,

$R_5$ représente l'hydrogène, un halogène, un groupe -N$R_{13}R_{14}$, alkyle en $C_1$-$C_3$, halogénoalkyle en $C_1$-$C_3$, alcoxy en $C_1$-$C_3$ ou halogénoalcoxy en $C_1$-$C_2$,

$R_6$ représente l'hydrogène, un groupe alkyle en $C_1$-$C_3$ ou alcoxy en $C_1$-$C_3$,

X représente l'oxygène ou le soufre, et

E représente l'azote ou le groupe méthine,

$R_7$ représente un groupe alkyle en $C_1$-$C_4$ ou alcényle en $C_3$-$C_5$ éventuellement substitué par des halogènes ou des groupes alcoxy en $C_1$-$C_3$,

$R_8$ représente un groupe alkyle en $C_1$-$C_4$, halogénoalkyle en $C_1$-$C_4$ ou alcoxyalkyle en $C_2$-$C_6$,

$R_9$, $R_{10}$, $R_{13}$ et $R_{14}$ représentent chacun, indépendamment les uns des autres, l'hydrogène ou un groupe alkyle en $C_1$-$C_3$,

Q représente l'oxygène, le soufre, un pont sulfinyle ou sulfonyle, et

n est égal à 0 ou 1,

et les sels de ces composés.

2. Composés selon la revendication 1, caracterisés en ce que X représente l'oxygène.

3. Composés selon la revendication 1, caractérisés en ce que $R_4$ et $R_5$, ensemble, ne contiennent pas plus de 4 atomes de carbone.

4. Composés selon la revendication 1, caractérisés en ce que $R_6$ représente l'hydrogène.

5. Composés selon la revendication 1, caractérisés en ce que $R_1$ et $R_2$ représentent l'hydrogène.

6. Composés selon la revendication 1, caractérisés en ce que $R_3$ est en position 2 par rapport au groupe sulfonyle.

7. Composés selon la revendication 1, caractérisés en ce que le substituant $R_3$ contient 2 à 4 atomes de carbone.

8. Composés selon la revendication 1, caractérisés en ce que X représente l'oxygène et $R_1$, $R_2$ et $R_6$ l'hydrogène, $R_4$ et $R_5$, ensemble, ne contiennent pas plus de 4 atomes de carbone, et $R_3$ est en position 2 par rapport au groupe sulfonyle et contient de 2 à 4 atomes de carbone.

9. Composés selon la revendication 1, choisis dans le groupe formé par la
N-[2-(3,3,3-trifluoro-1-propène-1-yl)-phényl-sulfonyl]-N'-(4-méthoxy-6-méthyl-1,3,5-triazine-2-yl)-urée et la
N-[2-(3 3 3-trifluoro-1-propène-1-yl)-phényl-sulfonyl]-N'-(4,6-diméthoxy-1,3,5-triazine-2-yl)-urée.

10. Procédé de préparation des composés de formule I, revendication 1, caractérisé en ce que l'on fait réagir un phénylsulfonamide de formule II

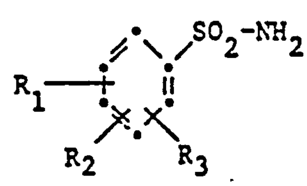

(II),

dans laquelle $R_1$, $R_2$ et $R_3$ ont les significations indiquées en référence à la formule I dans la revendication 1, en présence d'une base, avec un N-pyrimidinyl- ou -triazinyl-carbamate de formule III

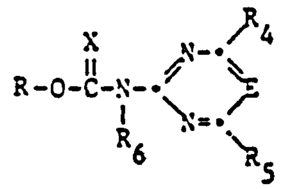 (III),

dans laquelle E, $R_4$, $R_5$, $R_6$ et X ont les significations indiquées en référence à la formule I dans la revendication 1 et R représente un groupe phényle, alkyle ou phényle substitué, et, le cas échéant, on convertit les composés obtenus en leurs sels.

11. Procédé de préparation des composés de formule I, revendication 1, caractérisé en ce que l'on fait réagir un phénylsulfonylisocyanate ou -isothiocyanate de formule IV

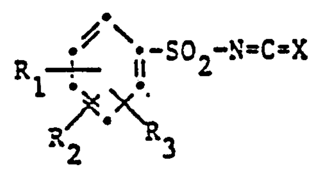 (IV),

dans laquelle $R_1$, $R_2$, $R_3$ et X ont les significations indiquées en référence à la formule I dans la revendication 1, éventuellement en présence d'une base, avec une amine de formule V

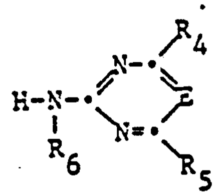 (V),

dans laquelle E, $R_4$, $R_5$ et $R_6$ ont les significations indiquées en référence à la formule I dans la revendication 1 et, le cas échéant, on convertit les composés obtenus en leurs sels.

12. Procédé de préparation des composés de formule I, revendication 1, caractérisé en ce que l'on fait réagir des sulfonamides de formule II ci-dessus, éventuellement en présence d'une base, avec un isocyanate ou isothiocyanate de formule VI

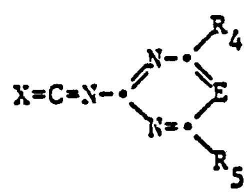 (VI),

dans laquelle E, $R_4$, $R_5$ et X ont les significations indiquées en référence à la formule I dans la revendication 1 et, le cas échéant, on convertit les composés obtenus en leurs sels.

13. Procédé de préparation des composés de formule I, revendication 1, caractérisé en ce que l'on fait réagir un N-phénylsulfonylcarbamate de formule VII

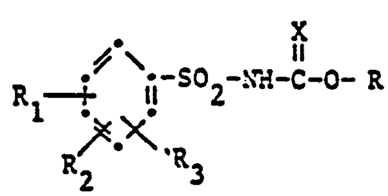 (VII),

dans laquelle $R_1$, $R_2$, $R_3$ et X ont les significations indiquées en référence à la formule I dans la revendication 1 et R représente un groupe phényle, alkyle ou phényle substitué, avec une amine de formule V donnée dans la revendication 11, et le cas échéant, on convertit les composés obtenus en leurs sels.

14. Procédé de préparation des sels des composes de formule I formés par addition selon l'une des revendications 10 à 13, caractérisé en ce que l'on fait réagir une sulfonylurée de formule I avec une amine, un hydroxyde de métal alcalin ou alcalino-terreux ou une base d'ammonium quaternaire.

15. Un agent herbicide et régulateur de la croissance des végétaux, caractérisé en ce qu'il contient en tant que substance active, avec des véhicules et/ou d'autres additifs, au moins une N-phénylsulfonyl-N'-triazinyl- ou -pyrimidinyl-urée de formule I, revendication 1.

16. L'utilisation des N-phénylsulfonyl-N'-triazinyl- ou -pyrimidinyl-urées de formule I, revendication 1, ou de produits en contenant, pour la lutte contre les croissances de végétaux indésirables.

17. L'utilisation des N-phénylsulfonyl-N'-triazinyl- ou -pyrimidinyl-urées de formule I, revendication 1, ou de produits en contenant, pour la régulation de la croissance des végétaux.

18. L'utilisation des N-phénylsulfonyl-N'-triazinyl- ou -pyrimidinyl-urées de formule I, revendication 1, ou de produits en contenant, pour la régulation de la croissance des végétaux cultivés en vue d'une augmentation de rendement.

19. L'utilisation des N-phénylsulfonyl-N'-triazinyl- ou -pyrimidinyl-urées de formule I ou de produits en contenant selon la revendication 16, pour la lutte sélective en pré-levée ou en post-levée contre les végétaux adventices dans les cultures de végétaux utiles.

20. L'utilisation des composés de formule I ou de produits en contenant selon la revendication 17, dans les cultures de céréales, de maïs et de riz.

21. L'utilisation des composés de formule I ou de produits en contenant selon la revendication 18, dans les cultures de soja.

**Revendications** pour l'Etat Contractant: AT

1. Un agent herbicide et régulateur de croissance des végétaux, caractérisé en ce qu'il contient en tant que substance active, avec 1 à 99,9 % de véhicules et/ou d'autres additifs, au moins une N-phénylsulfonyl-N'-triazinyl- ou -pyrimidinyl-urée de formule I

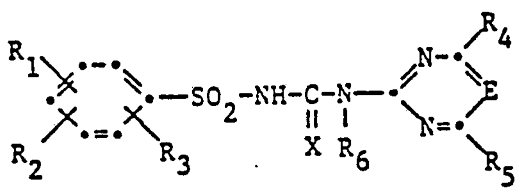

(I),

ou un sel d'un tel composé, à une concentration de 0,1 à 95 %,

$R_1$ represente l'hydrogène, un halogène, un groupe nitro, amino, alkyle en $C_1$-$C_5$, halogénoalkyle en $C_1$-$C_4$ ou un groupe -Q-$R_7$, -CO-$OR_8$ ou -$(CO)_n$-$NR_9R_{10}$,

$R_2$ représente l'hydrogène, un groupe alkyle en $C_1$-$C_4$, alcoxy en $C_1$-$C_4$, alkylthio en $C_1$-$C_4$, halogénoalkyle en $C_1$-$C_4$, halogénoalcoxy en $C_1$-$C_4$, un halogène ou un groupe alcoxyalkyle contenant au maximum 4 atomes de carbone,

$R_3$ représente un groupe alcényle en $C_2$-$C_{10}$ mono- ou poly-substitué par le fluor et dont la double liaison est reliée directement au noyau phényle,

$R_4$ représente un groupe alkyle en $C_1$-$C_3$, halogénoalkyle en $C_1$-$C_3$, alcoxy en $C_1$-$C_3$ ou halogénoalcoxy en $C_1$-$C_3$,

$R_5$ représente l'hydrogène, un halogène, un groupe -$NR_{13}R_{14}$, alkyle en $C_1$-$C_3$, halogénoalkyle en $C_1$-$C_3$, alcoxy en $C_1$-$C_3$ ou halogénoalcoxy en $C_1$-$C_2$,

$R_6$ représente l'hydrogène, un groupe alkyle en $C_1$-$C_3$ ou alcoxy en $C_1$-$C_3$,

X représente l'oxygène ou le soufre, et

E représente l'azote ou le groupe méthine,

$R_7$ représente un groupe alkyle en $C_1$-$C_4$ ou alcényle en $C_3$-$C_5$ éventuellement substitué par des halogènes ou des groupes alcoxy en $C_1$-$C_3$,

$R_8$ représente un groupe alkyle en $C_1$-$C_4$, halogénoalkyle en $C_1$-$C_4$ ou alcoxyalkyle en $C_2$-$C_6$,

$R_9$, $R_{10}$, $R_{13}$ et $R_{14}$ représentent chacun, indépendamment les uns des autres, l'hydrogène ou un groupe alkyle en $C_1$-$C_3$,

Q représente l'oxygène, le soufre, un pont sulfinyle ou sulfonyle, et

n est égal à 0 ou 1.

2. Agent selon la revendication 1, caractérisé en ce que X représente l'oxygène.

3. Agent selon la revendication 1, caractérisé en ce que $R_4$ et $R_5$, ensemble, ne contiennent pas plus de 4 atomes de carbone.

4. Agent selon la revendication 1, caractérisé en ce que $R_6$ représente l'hydrogène.

5. Agent selon la revendication 1, caractérisé en ce que $R_1$ et $R_2$ représentent l'hydrogène.

6. Agent selon la revendication 1, caractérisé en ce que $R_3$ est en position 2 par rapport au groupe sulfonyle.

7. Agent selon la revendication 1, caractérisé en ce que le substituant $R_3$ contient 1 à 4 atomes de carbone.

8. Agent selon la revendication 1, caractérisé en ce que X représente l'oxygène et $R_1$, $R_2$ et $R_6$ l'hydrogène, $R_4$ et $R_5$, ensemble, ne contiennent pas plus de 4 atomes de carbone, et $R_3$ occupe la position 2 par rapport au groupe sulfonyle et contient de 2 à 4 atomes de carbone.

9. Agent selon la revendication 1, contenant une substance active choisie dans le groupe formé par:
la N-[2-(3,3,3-trifluoro-1-propène-1-yl)-phényl-sulfonyl]-N'-(4-méthoxy-6-méthyl-1,3,5-triazine-2-yl)-urée, et

la N-[2-(3,3,3-trifluoro-1-propène-1-yl)-phényl-sulfonyl]-N'-(4,6-diméthoxy-1,3,5-triazine-2-yl)-urée.

10. Procédé de préparation des composés de formule I, revendication 1, caractérisé en ce que:

a) on fait réagir un phénylsulfonamide de formule II

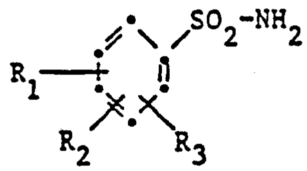

(II),

dans laquelle $R_1$, $R_2$ et $R_3$ ont les significations indiquées en référence à la formule I, revendication 1, en présence d'une base, avec un N-pyrimidinyl- ou -triazinylcarbamate de formule III

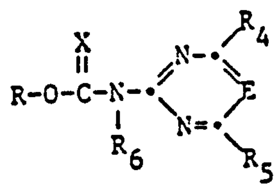

(III),

dans laquelle E, $R_4$, $R_5$, $R_6$ et X ont les significations indiquées en référence à la formule I, revendication 1, et R représente un groupe phényle, alkyle ou phényle substitué, ou bien

b) on fait réagir un phénylsulfonylisocyanate ou -isothiocyanate de formule IV

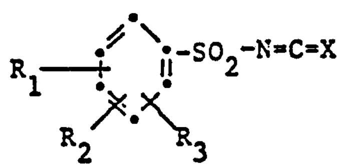

(IV),

dans laquelle $R_1$, $R_2$, $R_3$ et X ont les significations indiquées en référence à la formule I, revendication 1, eventuellement en présence d'une base, avec une amine de formule V

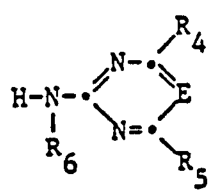

(V),

dans laquelle E, $R_4$, $R_5$ et $R_6$ ont les significations indiquées en référence à la formule I, revendication 1, ou bien

c) on fait réagir des sulfonamides de formule II ci-dessus, éventuellement en présence d'une base, avec un isocyanate ou isothiocyanate de formule VI

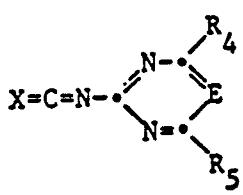

(VI),

dans laquelle E, $R_4$, $R_5$ et X ont les significations indiquées en référence à la formule I revendication 1 ou bien

d) on fait réagir un N-phénylsulfonylcarbamate de formule VII

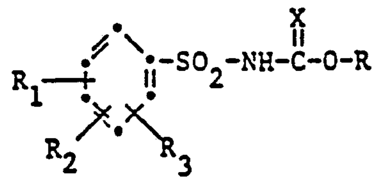

(VII),

dans laquelle $R_1$, $R_2$, $R_3$ et X ont les significations indiquées en référence à la formule I, revendication 1, et R représente un groupe phényle, alkyle ou phényle substitué, avec une amine de formule V ci-dessus et, le cas échéant, on convertit les composés obtenus en leurs sels, par réaction d'une sulfonylurée de formule I avec une amine, un hydroxyde de métal alcalin ou alcalino-terreux ou une base d'ammonium quaternaire.

11. L'utilisation des N-phénylsulfonyl-N'-triazinyl- ou -pyrimidinyl-urées de formule I, revendication 1, ou de produits en contenant pour la lutte contre les croissances de végétaux nuisibles.

12. L'utilisation des N-phénylsulfonyl-N'-triazinyl- ou -pyrimidinyl-urées de formule I, revendication 1, ou de produits en contenant, pour la régulation de la croissance des végétaux.

13. L'utilisation des N-phénylsulfonyl-N'-triazinyl- ou -pyrimidinyl-urées de formule I, revendication 1, ou de produits en contenant, pour la régulation de croissance des végétaux cultivés en vue d'une augmentation de rendement.

14. L'utilisation des N-phénylsulfonyl-N'-triazinyl- ou -pyrimidinyl-urées de formule I, revendication 11, ou de produits en contenant, pour la lutte sélective en pré-levée ou en post-levée contre les végétaux adventices dans les cultures de végétaux utiles.

15. L'utilisation des composés de formule I ou de produits en contenant selon la revendication 12, dans les cultures de céréales, de maïs ou de riz.

16. L'utilisation des composés de formule I ou de produits en contenant selon la revendication 13, dans les cultures de soja.